# EUROPEAN PATENT SPECIFICATION

(11) **EP 4 629 995 B1**
(45) Date of publication and mention of the grant of the patent: **11.11.2026**
(21) Application number: 23844268.5
(22) Date of filing: 08.12.2023
(51) Int. Cl.: A61K 31/4745, A61K 31/5383, A61K 31/404, A61K 31/436, A61K 31/502, A61K 31/517, A61K 31/138, A61K 45/06, A61P 35/00

(54) **COMBINATION THERAPY WITH AN ANALOG OF BLEBBISTATIN FOR THE TREATMENT OF CANCER**
KOMBINATIONSTHERAPIE MIT EINEM BLEBBISTATIN-ANALOG ZUR BEHANDLUNG VON KREBS
POLYTHÉRAPIE UTILISANT UN ANALOGUE DE LA BLEBBISTATINE POUR LE TRAITEMENT DU CANCER

(30) Priority: 08.12.2022 US 202263431234 P
(43) Date of publication of application: 15.10.2025
(73) Proprietor: The University of Florida Research Foundation, Inc., Gainesville, Florida 32611 (US); Mayo Foundation for Medical Education and Research, Rochester, Minnesota 55905 (US)
(72) Inventor: MILLER, Courtney Anne, Jupiter, Florida 33477 (US); ROSENFELD, Steven S., Vedra Beach, Florida 32082 (US); GRIFFIN, Patrick Robert, Jupiter, Florida 33458 (US); KAMENECKA, Theodore Mark, Palm Beach Gardens, Florida 33410 (US)
(74) Representative: Secerna LLP
(86) International application number: PCT/US2023/083143
(87) International publication number: WO 2024/124147

(56) References cited:
- WO-A1-2019/241469
- SIGRID VERHASSELT ET AL: "Improved synthesis and comparative analysis of the tool properties of new and existing D-ring modified ( S )-blebbistatin analogs", EUROPEAN JOURNAL OF MEDICINAL CHEMISTRY, vol. 136, 18 August 2017 (2017-08-18), AMSTERDAM, NL, pages 85 - 103, XP055763936, ISSN: 0223-5234, DOI: 10.1016/j.ejmech.2017.04.072
- SINGH DEEPIKA: "Current updates and future perspectives on the management of renal cell carcinoma", LIFE SCIENCE, PERGAMON PRESS, OXFORD, GB, vol. 264, 25 October 2020 (2020-10-25), XP086413464, ISSN: 0024-3205, [retrieved on 20201025], DOI: 10.1016/J.LFS.2020.118632

## Description

This application claims priority to U. S. Patent Application Serial Number 63/431,234, filed December 8, 2022.

### STATEMENT OF GOVERNMENT SUPPORT

This invention was made with government support under Grant No. R01NS118513 awarded by the National Institutes of Health. The government has certain rights in the invention.

### BACKGROUND

Cell migration is an essential step of cancer metastasis and cell motility mechanisms rely on the activity of actomyosin networks in the cell. The actin cytoskeleton regulates cell polarity and adhesion, facilitating cells transition towards malignant phenotype including invasion of adjacent tissues, and metastasis. The major cellular actin-binding molecular motor proteins, nonmuscle myosin IIs, regulate and mechanically change the microenvironment during cancer cell migration and tumor proliferation. Members of the myosin superfamily can act as either enhancers or suppressors of tumor progression where inactivation or upregulation of myosin is associated with malignant phenotype, cancer cell migration, and metastasis. Interestingly, knockdown of NMIIs results in altered expression of genes associated with epithelial-to-mesenchymal transition (EMT), angiogenesis, and senescence (Halder D. et al., Nonmuscle myosin IIA and IIB differentially modulate migration and alter gene expression in primary mouse tumorigenic cells. Molecular Biology of the Cell, 2019. 30(12): 1463-1476). Taken together, targeting nonmuscle myosin IIs in cancer represents an important strategy to treat various cancers by reducing proliferation and metastasis (Naydenov NG, et al. Myosin Motors: Novel Regulators and Therapeutic Targets in Colorectal Cancer. Cancers. 2021. 13(4):741; Z-K. Xia, et al., Nonmuscle myosin IIa is associated with poor prognosis of esophageal squamous cancer, Diseases of the Esophagus. 2012. (25)(5): 427-436; Li Y., et al. Myosins as fundamental components during tumorigenesis: diverse and indispensable. Oncotarget. 2016; 7: 46785-46812).

Glioblastoma (GBM) is the most common and lethal of primary, glial tumors and defies therapies that are successful against other solid malignancies (Puduvalli, V.K. et al., Chemotherapy of High-Grade Astrocytomas in Adults. Prog Neurol Surg, 2018. 31: p. 116-144; Verhaak, R.G., et al., Integrated genomic analysis identifies clinically relevant subtypes of glioblastoma characterized by abnormalities in PDGFRA, IDH1, EGFR, and NF1. Cancer Cell, 2010. 17(1): p. 98-110; Yuan, J., et al., Single-cell transcriptome analysis of lineage diversity in high-grade glioma. Genome Med, 2018. 10(1): p. 57; Neftel, C., et al., An Integrative Model of Cellular States, Plasticity, and Genetics for Glioblastoma. Cell, 2019. 178(4): p. 835-849 e21; Wang, L.B., et al., Proteogenomic and metabolomic characterization of human glioblastoma. Cancer Cell, 2021. 39(4): p. 509-528 e20).Glioblastomas are aggressive and malignant brain tumors representing nearly half of all malignant brain tumors and that originate from the glial cells of the brain (NORD Rare Disease Report, Glioblastoma, last updated 30 Oct 2023, accessed at https://rarediseases.org/rare-diseases/glioblastoma-multiforme/). Glioblastoma is the most common primary malignant brain cancer in adults with a 5-year overall survival rate of between ≤5% to 7.2% after diagnosis (Batash, R. N., et al., Glioblastoma Multiforme, Diagnosis and Treatment; Recent Literature Review. Curr Med Chem_2017. 24(27):3002-3009; Chien, L.N. et al., Comparative Brain and Central Nervous System Tumor Incidence and Survival between the United States and Taiwan Based on Population-Based Registry. Front Public Health, 2016. 4:151; Ding, Z. Z., et al., Leukemia-Associated Rho Guanine Nucleotide Exchange Factor and Ras Homolog Family Member C Play a Role in Glioblastoma Cell Invasion and Resistance. Am J Pathol, 2020. 190(10):2165-2176; Wu, W., et al., Glioblastoma multiforme (GBM): An overview of current therapies and mechanisms of resistance. Pharmacol Res, 2021. 171: 105780; Ostrom, Q. T., et al., CBTRUS Statistical Report: Primary Brain and Other Central Nervous System Tumors Diagnosed in the United States in 2014-2018. Neuro Oncol, 2021. 23(12 Suppl 2):iii1-iii105). The standard of care includes maximal surgical resection followed by radiation and temozolomide (TMZ), an alkylating chemotherapeutic (Rahman, M. I., et al., Selective Vulnerability of Senescent Glioblastoma Cells to BCL-XL Inhibition. Mol Cancer Res, 2022. 20(6):938-948), and sometimes the use of tumor-treating fields (TTFs). Because of the high rate of recurrence and invasive nature of GBM, however, this care only extends survival following initial diagnosis to approximately one year. Hence, despite decades of research, no cure has been identified, and so GBM remains an ultimately lethal disease.

Numerous factors contribute to treatment failure in GBM. These include the limited ability to resect tumor because of the extensive invasion of individual tumor cells into the adjacent normal brain, the significant radiation resistance of these invading tumor cells, and over half of all GBM patients being non-responsive to TMZ due to their genetic and epigenetic status (IDH wildtype and MGMT unmethylated) (Ding et al., 2020; Kamson, D.O., et al., The Role of Temozolomide in Patients With Newly Diagnosed Wild-Type IDH, Unmethylated MGMTp Glioblastoma During the COVID-19 Pandemic. JAMA Oncol. 2021;7(5):675-676). Further, oncogenic kinases in GBM are frequently redundant, enabling cells to overcome blockade of one by activating or upregulating another to bypass the blockade (Ivkovic, S., et al., Direct inhibition of myosin II effectively blocks glioma invasion in the presence of multiple motogens. Mol Biol Cell, 2012. 23(4): p. 533-42). Also, GBM is an immunosuppressive tumor, evading the immune response through a variety of mechanisms that constitute a roadblock to effective tumor vaccines (Frederico, S.C., *et al.,* Making a Cold Tumor Hot: The Role of Vaccines in the Treatment of Glioblastoma. *Front Oncol,* 2021. 11: p. 672508). In addition, although the blood brain barrier (BBB) is leaky in someregions of a GBM, invasive tumor cells can be found within normal brain and are surrounded by an intact BBB that protects them from CNS-impermeant therapeutics. GBM tumors contain subpopulations of cells with stem cell-like features that resist DNA-damaging therapies, leading to inevitable recurrence and fatality (Bao, S., et al., Glioma stem cells promote radioresistance by preferential activation of the DNA damage response. Nature, 2006. 444(7120): p. 756-60). In addition, the two defining phenotypes of GBM are invasion and proliferation, also known as Go and Grow: GBM cells do only one or the other. However, blocking one stimulates the other.

Patients with recurrent high-grade glioma have an exceptionally poor prognosis -- if untreated, GBM is fatal typically within three months of diagnosis - and there have been no new drugs for these patients since 2008, when bevacizumab was approved. Survival rates for patients with GBM have shown no notable improvement in population statistics in the last three decades (Tamimi, A.F. and Juweid, M., Epidemiology and Outcome of Glioblastoma. In: De Vleeschouwer S, editor. Glioblastoma [Internet]. Brisbane (AU): Codon Publications; 2017 Sep 27. Chapter 8. PMID: 29251870).

More than 14,490 individuals are expected to receive a GBM diagnosis in 2023, according to the National Brain Tumor Society. The incidence of GBM is reported to be as high as 3.3 individuals per 100,000 in the US with a duration of 1.2 years, indicating approximately 13,264 patients with GBM currently in the US, well below the threshold of 200,000 cases defining the epidemiological criterion of an orphan disease.

Patients with GBM present with two types of symptoms, namely generalized and focal (NORD Glioblastoma report). Focal symptoms are dependent on the location and size of the tumor; for example, if the tumor is in eloquent brain, the patient may have more issues speaking or understanding speech (NORD Glioblastoma report). However, common symptoms according to the NORD GlioblastomaReport and AANS.org include, headaches, seizures, nausea/vomiting, confusion, memory loss, muscle weakness, visual changes, double or blurred vision, language deficits, cognitive changes, and decrease in normal function.

Individuals suspected of having a GBM typically undergo a full physical and neurological examination. The neurological examination assesses patient sensory and muscle responses (NORD Glioblastoma report). A contrast-enhanced magnetic resonance imaging (MRI) is often used to identify GBMs; however, a tissue biopsy is required to make any definite diagnosis (NORD Glioblastoma report). GBMs can be located anywhere in the brain and do not regularly spread outside of the brain (NORD Glioblastoma report). GBMs tend to affect older individuals (age 45 to 70) with rare occurrences in children (NORD Glioblastoma report). The average age of diagnosis is 64 years of age with a slightly higher rate in men than women (NORD Glioblastoma report). Caucasians have the highest rate of GBM diagnoses compared to other ethnic groups such as African Americans, Asians, and Native Americans (NORD Glioblastoma report).

The World Health Organization (WHO) classifies GBMs into four main categories: glioblastoma isocitrate dehydrogenase (IDH)-mutant, glioblastoma IDH-wildtype, glioblastoma NOS (not otherwise specified), and Not-elsewhere-classified (NEC) glioblastoma as further detailed in Table 1 (Grochans, S. A., et al., Epidemiology of Glioblastoma Multiforme-Literature Review. Cancers (Basel), 2022. 14(10):2412).

**Table 1: WHO Classification of GBMs into Four Main Categories**

| **WHO Classification of GBMs** | **Description** |
|---|---|
| Glioblastoma, isocitrate dehydrogenase (IDH) wildtype | Approximately 90% of cases, developing de novo at about 60 years of age |
| Glioblastoma, IDH-mutant | Approximately 10% of cases, secondary GBM that usually develops in younger patients with gliomas of higher differentiation (WHO grades I-III); it carries a significantly better prognosis than wildtype IDH |
| Glioblastoma not otherwise specified (NOS) | The IDH mutation status could not be determined due to a lack of histological or molecular material for testing |
| Not-elsewhere-classified (NEC) Glioblastoma | This fourth category distinguished in recent years. The necessary determinations regarding the classification of the tumor have been made, but the results do not allow matching the tumor to any of the aforementioned categories of the 2016 WHO division. This situation may occur in the case of discrepancies between the clinical, histological, immunohistological, and genetic features of the tumor. There is also a possibility that a GBM subunit exists with an unknown combination of features that is not yet classified in the WHO division. |

| | |
|---|---|
| WHO = World Health Organization; IDH = isocitrate dehydrogenase; NOS = not otherwise specified; NEC = Not-elsewhere classified | |

Karnofsky performance status (KPS) is used to determine functional capacity, where a score of 100 indicates that the patient can perform all tasks normally with minor signs of disease while a score of 50 indicates that the patient requires considerable help in everyday life (NORD Glioblastoma report). O-6 methylguanine DNA methyltransferase (MGMT) is an enzyme responsible for DNA repair, and patients with GBM will often undergo methylation profiling to determine MGMT status because it can influence the effectiveness of chemotherapy (NORD Glioblastoma report). Individuals with a normal form (methylated or partially methylated) of MGMT tend to fare better during and after chemotherapy (NORD Glioblastoma report). Further, individuals that are both IDH wildtype and MGMT unmethylated appear to receive no benefit from TMZ, which has quality of life and immunosuppressive effects (Kamson and Grossman, 2021; Torre, M. P. Y., et al., The predictive value of partial MGMT promoter methylation for IDH-wild-type glioblastoma patients, Neuro-Oncology Practice, 2023. 10(2): 126-131). As a result, there is observed a shift away from TMZ treatment in this group, which constitutes over half of all GBM cases.

As summarized above, standard treatment for primary GBM includes maximal safe surgical resection followed by radiation and TMZ, an alkylating chemotherapeutic (Rahman et al. 2022); treatment methods may also include TTF that are alternating electric fields therapy to prevent cancer cells from multiplying (NORD Glioblastoma report). Bevacizumab and Gliadel wafers are FDA-approved chemotherapy agents for the treatment of GBM but have shown limited success (NORD Glioblastoma report).

GBM is one of the most complex, deadly, and treatment-resistant cancers (National Brain Tumor Society). Underscoring a significant unmet medical need for treatment of GBM is the 5-year survival rate of less than about 5% (Batash et al. 2017; Chien et al., 2016; Ostrom et al., 2021), and there has not been any notable improvements in survival rates for decades (Tamimi and Juweid, 2017; National Brain Tumor Society). The National Brain Tumor Society estimates that more than 10,000 individuals in the US succumb to GBM every year.

The average survival time for patients with GBM who have undergone combination treatments of surgery, chemotherapy, and radiation is 14.6 months (NORD Glioblastoma report). Individuals with GBM IDH-mutant protein have a higher overall survival rate than those with GBM IDH-wildtype protein (NORD Glioblastoma report).

The nonmuscle myosin II (NMII) family of molecular motors are irreplaceable components of the machinery that drives the invasive phenotype of tumor cell metastasis. They also play an important role in mitosis, which drives tumor proliferation (Picariello, H. S., et al. Myosin IIA suppresses glioblastoma development in a mechanically sensitive manner. Proc Natl Acad Sci U S A, 2019. 116(31): 15550-15559; Ivkovic, S., et al. Direct Inhibition of myosin II effectively blocks glioma invasion in the presence of multiple mitogens. Mol Biol Cell, 2012. 23(4): 533-42; Beadle, C., et al. The role of myosin II in glioma invasion of the brain. Mol Biol Cell, 2008. 19(8): 3357-68). In GBM, for example, inhibiting NMIIA and/or NMIIB effectively blocks GBM dispersion, regardless of the activity upstream and invasion stimulating receptor tyrosine kinases (RTKs). Cytokinesis, which is the last stage of mitosis, is blocked by deleting NMIIA and IIB together, and this has been shown to significantly prolong survival in genetically engineered mouse models (GEMMs) of GBM. Specifically, extensive genetic interventions have shown that simultaneous disruption of two non-muscle myosin II (NMII) molecular motors (NMIIA and IIB) meet the criteria of blocking GBM invasion and proliferation. In addition, NMII plays other, non-canonical roles that are particularly relevant to cancer biology, including 1) regulation of ERK and SRC signaling and integrin and RTK function (Picariello, H. S., et al. Myosin IIA suppresses glioblastoma development in a mechanically sensitive manner. Proc Natl Acad Sci U S A, 2019. 116(31): 15550-15559; Rai, V., et al. Myosin IIA Heavy Chain Phosphorylation Mediates Adhesion Maturation and Protrusion in Three Dimensions. J Biol Chem, 2017. 292(8): 3099-3111; Morin, N. A., et al. Nonmuscle myosin heavy chain IIA mediates integrin LFA-1 de-adhesion during T lymphocyte migration. J Exp Med, 2008. 205:195-205); 2) regulation of p53 function in squamous cell carcinoma (Schramek, D. et al. Direct in vivo RNAi screen unveils myosin IIa as a tumor suppressor of squamous cell carcinomas, Science, 2014. 343(6168):309-13); and 3) control of resistance to MEK/ERK inhibitors and of mitochondrial fission and reactive oxygen species formation in melanoma (Orgaz, J. L. et al. Myosin II Reactivation and Cytoskeletal Remodeling as a Hallmark and a Vulnerability in Melanoma Therapy Resistance. Cancer Cell, 2020. 37, 85-103).

However, the translational potential of this research has been limited by the lack of a clinically safe, CNS-penetrant NMII small molecule inhibitor. To this end, Compound 1, (S)-3a-hydroxy-6-methyl-1-(2-methylquinolin-6-yl)-1,2,3,3a-tetrahydro-4H-pyrrolo[2,3-b]quinolin-4-one: 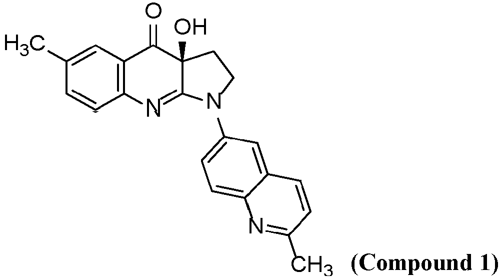 was identified as a well-tolerated, dual small molecule inhibitor of NMIIA and IIB with a high degree of brain penetrance (WO2019/241469), a requirement for an effective GBM therapeutic.

### SUMMARY

To overcome these challenges, the present disclosure provides methods for the treatment of a cancer in a subject suffering therefrom. In embodiments, the cancer is characterized by rapid tumor cell proliferation and aggressive invasion of surrounding tissues. The methods comprise administering to the subject a combination of Compound 1, or a pharmaceutically acceptable salt thereof: 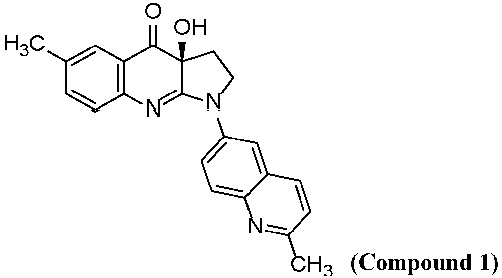 and at least one anti-cancer therapy.

Also provided as another embodiment is a combination of Compound 1, or a pharmaceutically acceptable salt thereof, and at least one anti-cancer therapy for the treatment of a cancer characterized by rapid tumor cell proliferation and aggressive invasion of surrounding tissues.

### BRIEF DESCRIPTION OF THE DRAWINGS

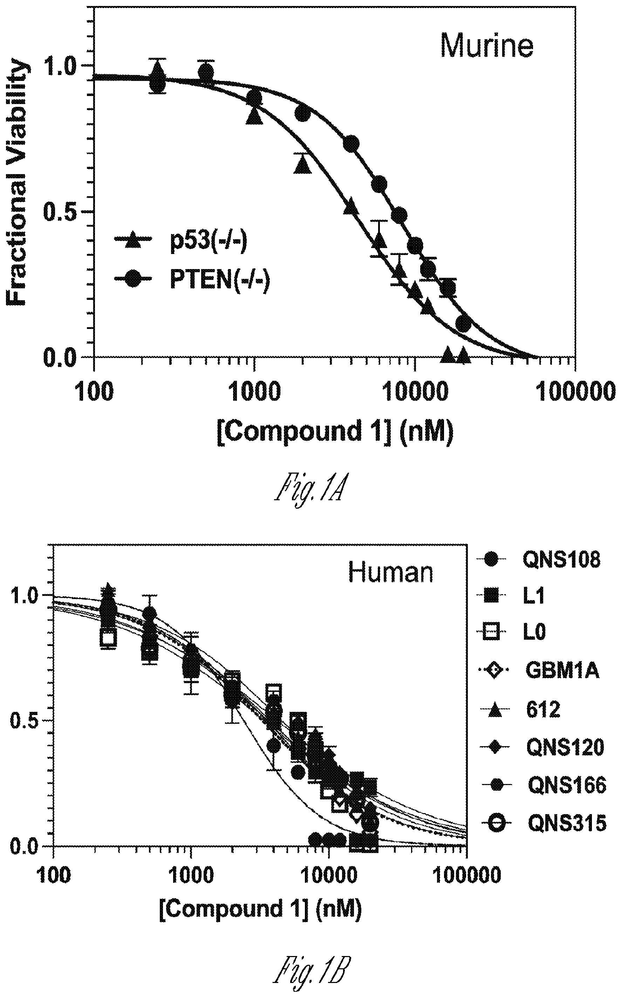

**FIG. 1A and FIG. 1B****.** Dose response curves of murine and human primary GBM cells to Compound 1. Murine proneural and mesenchymal GBM cell lines show similar sensitivities to Compound 1, with 4 - 6 µM EC₅₀ values (FIG. 1A). Corresponding dose response curves for 8 primary human GBM lines show similar potency and efficacy for Compound 1 (FIG. 1B).

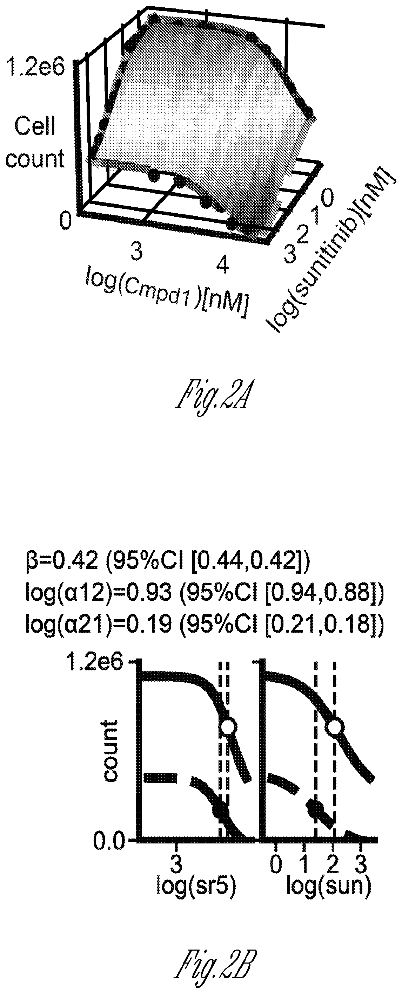

**FIG. 2A and FIG. 2B****.** A dose response surface generated by MuSyC shows synergistic interaction between Compound 1 and sunitinib (FIG. 2A). The synergistic dose range is indicated by the darker shading at the bottom right of the surface. Fitting the dose responses to the MuSyc algorithm defines synergies of efficacy (β) and potency (log(α12) and log (α21)) (FIG. 2B).

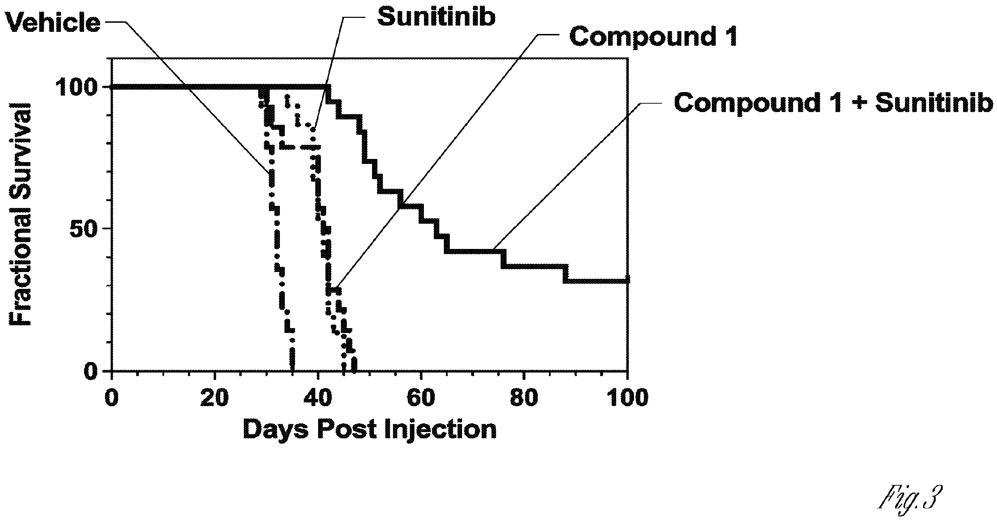

**FIG. 3****.** Kaplan Meier survival curves for *Trp53(-*/*-)* GEMMs treated with Compound 1, sunitinib, or a combination thereof. Mice were treated with vehicle (- × ×), Compound 1 (- - - -), sunitinib (× × × ×), or a combination of Compound 1 and sunitinib (-) until morbidity or 85 days post-retroviral injection, whichever came first. Compound 1 or sunitinib vs. vehicle = p<0.0001, log rank test with a 32% increase in median survival; sunitinib in combination with Compound 1 prolongs median survival more than two-fold, with 35% of mice surviving tumor-free at 100 days.

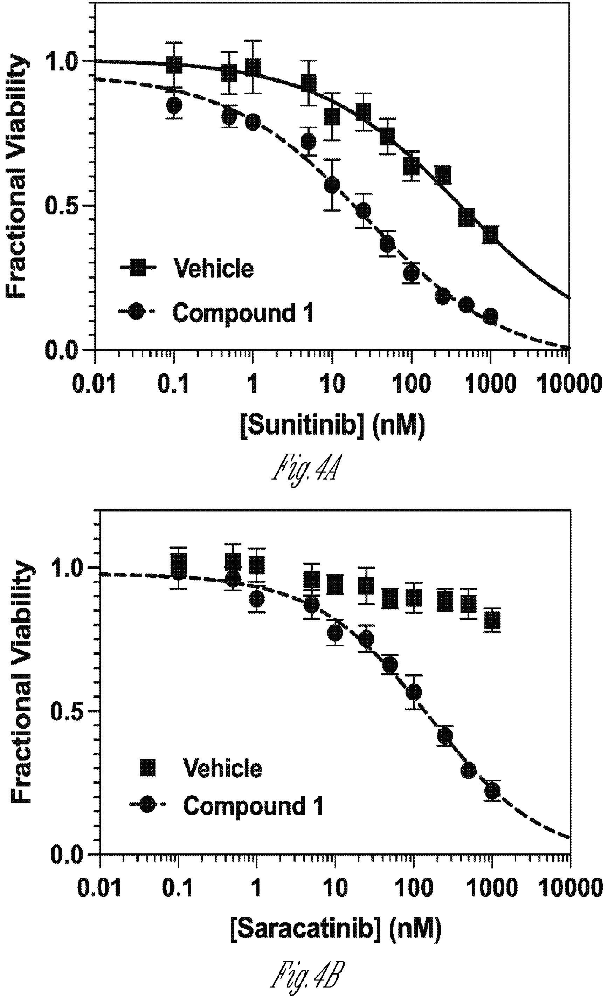

**FIG. 4A and FIG. 4B****.** *In vitro* dose responses (EC₅₀ curves) of Trp53-deleted (Trp53(-/-)) murine GBM cells to 5 µM Compound 1 in combination with sunitinib (FIG. 4A) or saracatinib (FIG. 4B) demonstrate synergism between Compound 1 and RTK inhibitor.

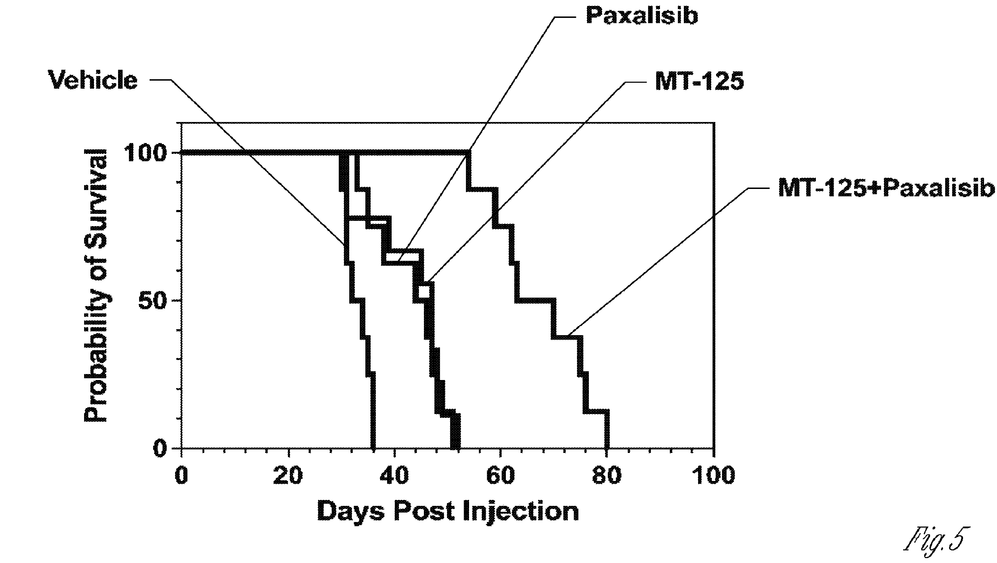

**FIG. 5****.** Paxalisib + Compound 1 *in vivo* synergy. Genetically engineered mice with floxed p53(-/-) alleles were injected with PDGF-IRES-cre retrovirus and treated with vehicle, paxalisib, Compound 1, or the combination of Compound 1 and paxalisib until morbidity. Differences between paxalisib or Compound 1 and vehicle and between the combination versus single agent Compound 1 or paxalisib are highly significant (p<0.0001, log rank test).

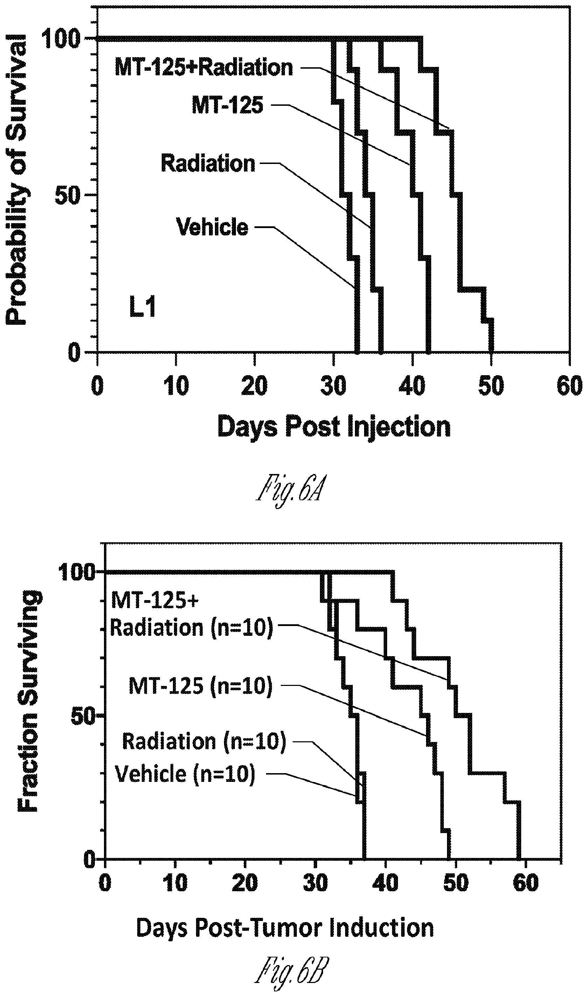

**FIG. 6A** and **FIG. 6B****.** Human PDX (FIG. 6A) or a p53-deleted GEMM (FIG. 6B) models were treated with daily doses of vehicle, Compound 1, radiotherapy, or a combination of Compound 1 and radiotherapy and then followed for survival. Differences between median survival for vehicle, Compound 1, radiation, and the combination were significant by a log rank test.

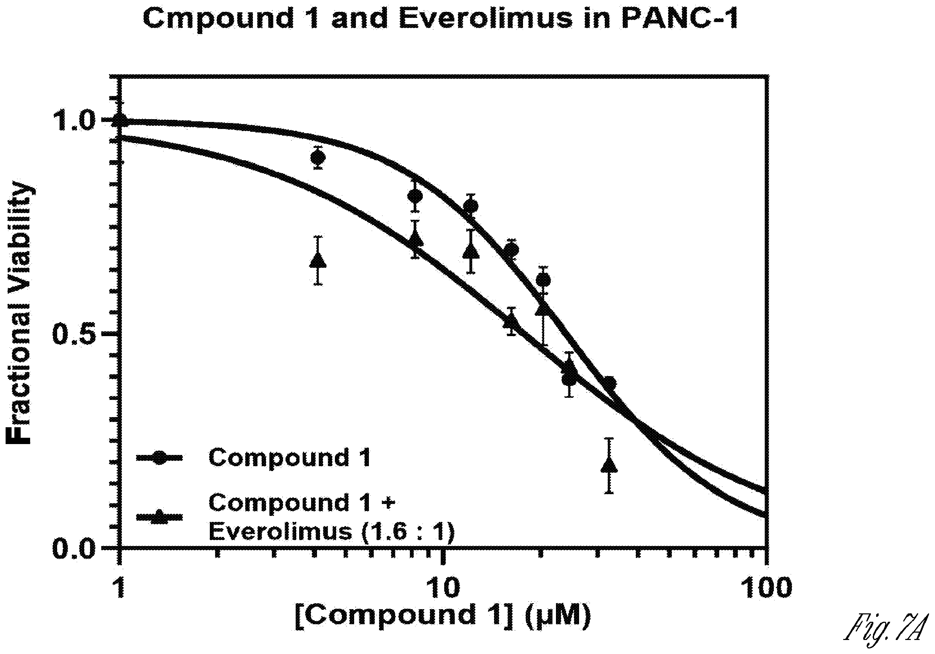

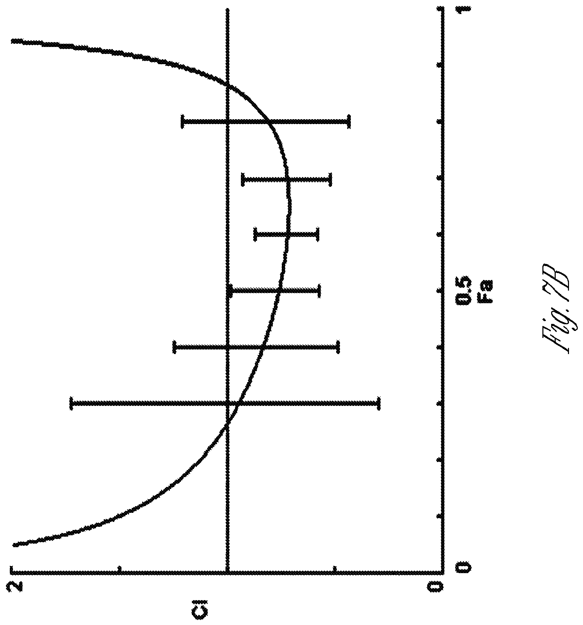

**FIG. 7A** **and** **FIG. 7B****.** Effect of Compound 1 alone and in combination with everolimus in pancreatic adenocarcinoma cells (PANC-1; FIG. 7A) a Combination Index (CI) plot of compound 1 and everolimus in PANC-1 showing synergism of the combination (FIG. 7B; Fa = function of effect level).

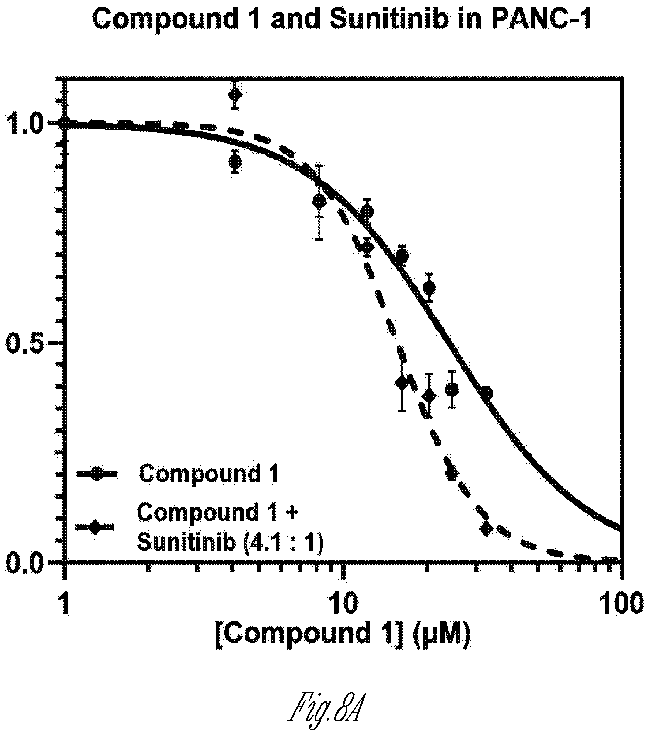

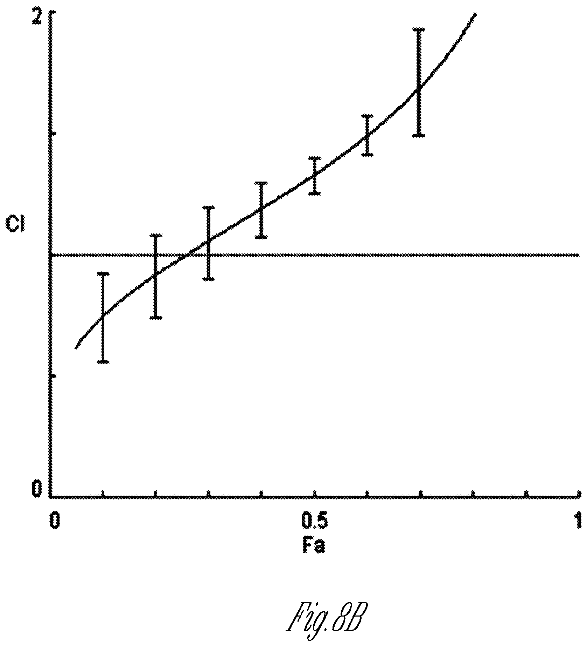

**FIG. 8A** **and** **FIG. 8B****.** Effect of Compound 1 alone and in combination with sunitinib in pancreatic adenocarcinoma cells (PANC-1; FIG. 8A) a Combination Index (CI) plot of compound 1 and sunitinib in PANC-1 showing synergism of the combination (FIG. 8B; Fa = function of effect level).

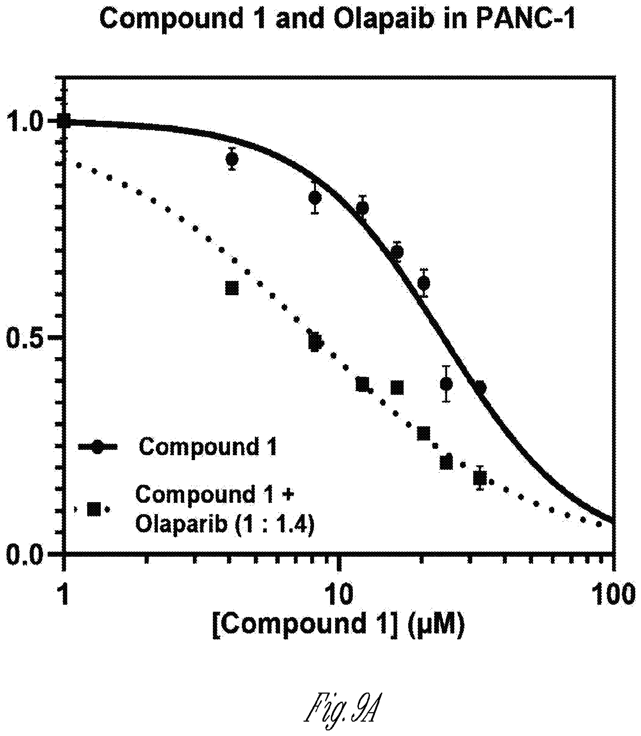

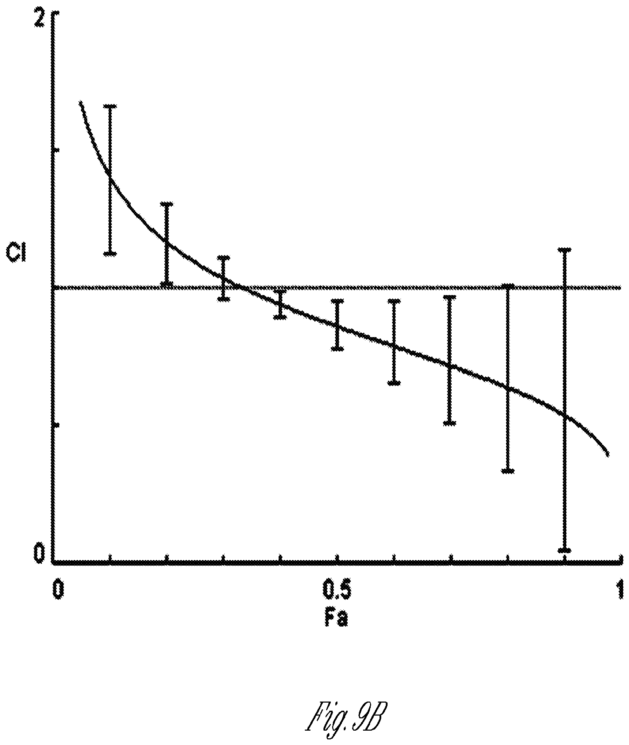

**FIG. 9A** **and** **FIG. 9B****.** Effect of Compound 1 alone and in combination with olaparib in pancreatic adenocarcinoma cells (PANC-1; FIG. 9A) a Combination Index (CI) plot of compound 1 and olaparib in PANC-1 showing synergism of the combination (FIG. 9B; Fa = function of effect level).

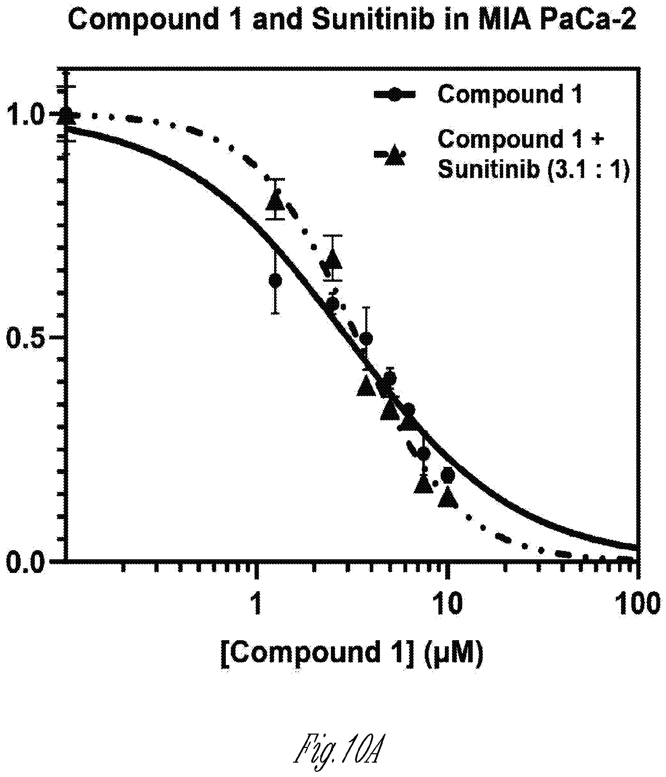

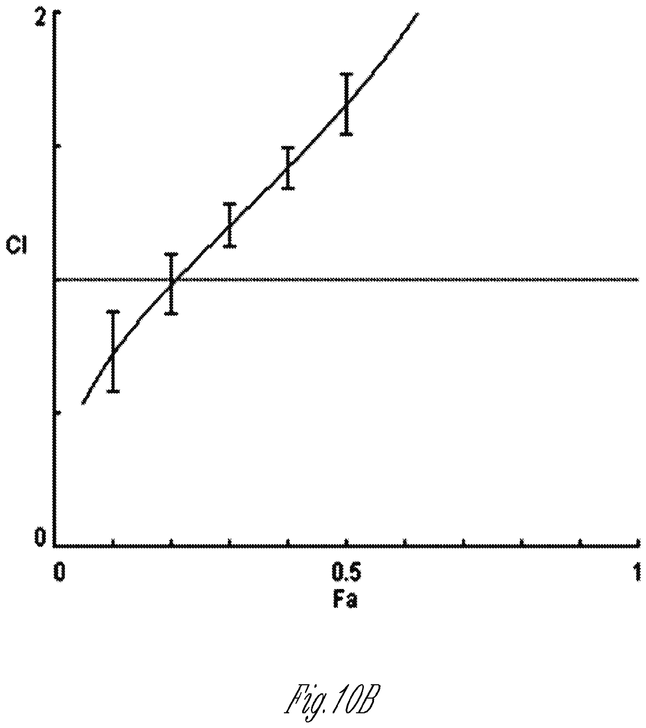

**FIG. 10A** **and** **FIG. 10B****.** Effect of Compound 1 alone and in combination with sunitinib in pancreatic adenocarcinoma cells (MIA PaCa-2; FIG. 10A) a Combination Index (CI) plot of compound 1 and sunitinib in PANC-1 showing synergism of the combination (FIG. 10B; Fa = function of effect level).

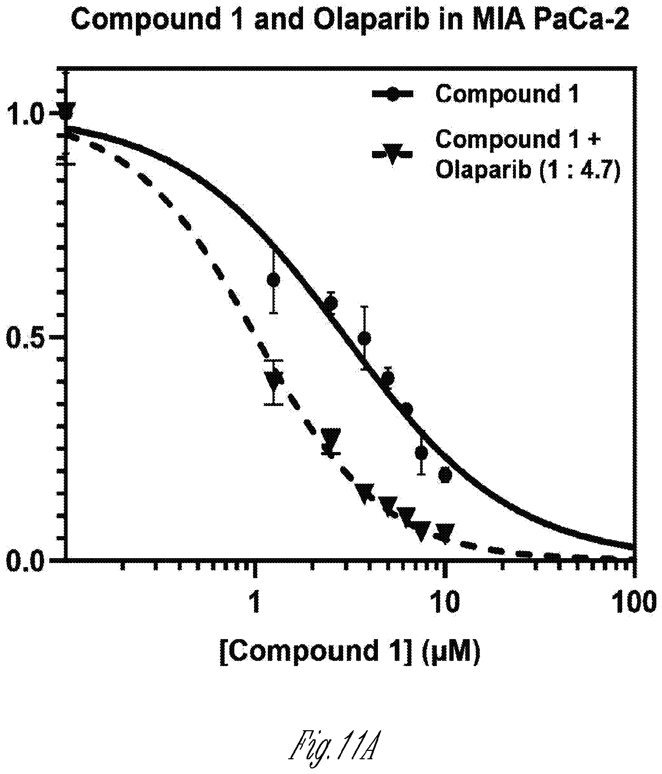

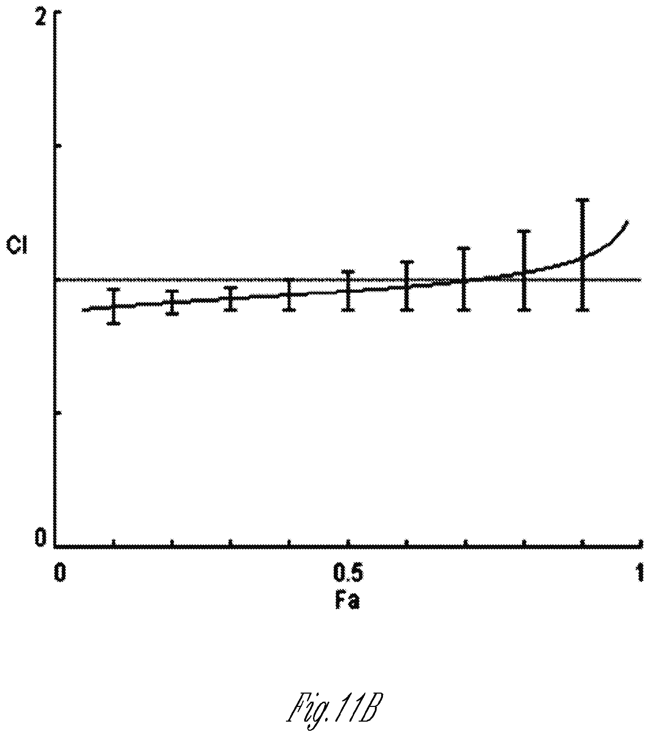

**FIG. 11A** **and** **FIG. 11B****.** Effect of Compound 1 alone and in combination with olaparib in pancreatic adenocarcinoma cells (MIA PaCa-2; FIG. 11A) a Combination Index (CI) plot of compound 1 and olaparib in PANC-1 showing synergism of the combination (FIG. 11B; Fa = function of effect level).

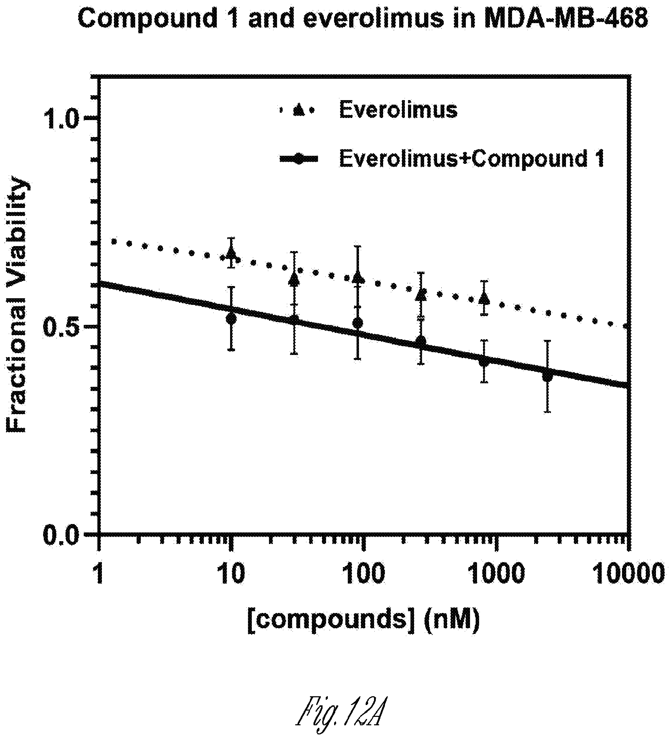

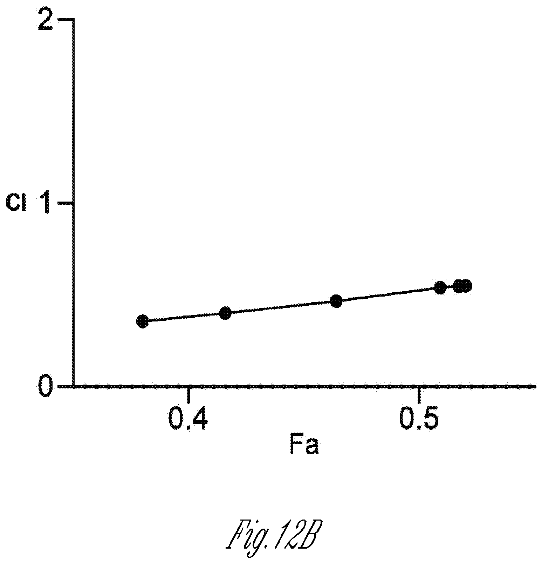

**FIG. 12A** **and** **FIG. 12B****.** Effect of Compound 1 alone and in combination with everolimus in TNBC cells (MDA-MB-468; FIG. 12A) a Combination Index (CI) plot of compound 1 and everolimus in MDA-MB-468 showing synergism of the combination (FIG. 12B; Fa = function of effect level).

### DETAILED DESCRIPTION

The present disclosure is premised upon the surprising synergism residing in a combination of Compound 1 and at least one anti-cancer therapy. The combination can extend cancer patient survival significantly beyond the sum of survivals achieved by Compound 1 and the anti-cancer therapy alone. As evidenced throughout various exemplary embodiments herein, the synergism residing in the combination also is reflected in patient survival even after cessation of therapy and tumor elimination therein.

Because NMII drives cancer cell growth, invasion-promoting signaling, and the DNA damage response, NMII is a compelling but relatively unexplored target for treatment of aggressive tumors, such as GBM. The present disclosure is premised, in part, on suppression of the two defining components, invasion and proliferation, respectively, of such tumors, of which GBM is an exemplary phenotype (Ivkovic, S., et al. (2012); Dhruv, H.D., et al., Reciprocal activation of transcription factors underlies the dichotomy between proliferation and invasion of glioma cells. PLoS One, 2013. 8(8): p. e72134; Picariello, H.S., et al (2019)). Inhibiting invasion stimulates proliferation and *vice versa* (Dhruv, H.D., et al (2013)), and so the most effective GBM therapy should block both. Settled research has demonstrated that the NMII family of molecular motors plays indispensable roles in both cell motility, which drives invasion, mitosis, and proliferation (Picariello, H.S., et al. (2019)). While targeting either NMIIA or IIB is sufficient to block GBM invasion, targeting both optimally blocks GBM proliferation (Ivkovic, S., et al. (2012); Picariello, H.S., et al. (2019)), implying that targeting of both NMIIA and IIB reduces proliferation and invasion regardless of how many oncogenic signaling pathways are activated, since NMII represents a point where these pathways converge (Ivkovic, S., et al. (2012); Picariello, H.S., et al. (2019); Kenchappa, R.S., et al., Myosin 10 Regulates Invasion, Mitosis, and Metabolic Signaling in Glioblastoma. iScience, 2020. 23(12): p. 101802; Beadle, C., et al. (2008)). Yet, little attention has been paid to targeting NMII therapeutically in light of the belief that such an NMII inhibitor would be toxic. However, as shown herein, Compound 1, a CNS-permeable small molecule inhibitor of both NMIIA and NMIIB, achieves therapeutic concentrations in mouse brain and is well-tolerated.

### Definitions

As used herein, and unless otherwise clear from context or specified to the contrary, the term "compound" is inclusive in that it encompasses a compound or a pharmaceutically acceptable salt thereof.

The singular forms "a," "an," and "the" include plural references, unless the context clearly dictates otherwise.

Some compounds described herein can exist in various isomeric forms, including configurational, geometric, and conformational isomers, including, for example, *cis-* or *trans-*conformations. The compounds may also exist in one or more tautomeric forms, including both single tautomers and mixtures of tautomers. The term "isomer" is intended to encompass all isomeric forms of a compound of this disclosure, including tautomeric forms of the compound. The compounds of the present disclosure may also exist in open-chain or cyclized forms. In some cases, one or more of the cyclized forms may result from the loss of water. The specific composition of the open-chain and cyclized forms may be dependent on how the compound is isolated, stored or administered. For example, the compound may exist primarily in an open-chained form under acidic conditions but cyclize under neutral conditions. All forms are included in the disclosure.

Some compounds described herein can have asymmetric centers and therefore exist in different enantiomeric and diastereomeric forms. A compound as described herein can be in the form of an optical isomer or a diastereomer. Accordingly, the disclosure encompasses compounds and their uses as described herein in the form of their optical isomers, diastereoisomers and mixtures thereof, including a racemic mixture. Optical isomers of the compounds of the disclosure can be obtained by known techniques such as asymmetric synthesis, chiral chromatography, simulated moving bed technology or via chemical separation of stereoisomers through the employment of optically active resolving agents.

Unless otherwise indicated, the term "stereoisomer" means one stereoisomer of a compound that is substantially free of other stereoisomers of that compound. Thus, a stereomerically pure compound having one chiral center will be substantially free of the opposite enantiomer of the compound. A stereomerically pure compound having two chiral centers will be substantially free of other diastereomers of the compound. A typical stereomerically pure compound comprises greater than about 80% by weight of one stereoisomer of the compound and less than about 20% by weight of other stereoisomers of the compound, for example greater than about 90% by weight of one stereoisomer of the compound and less than about 10% by weight of the other stereoisomers of the compound, or greater than about 95% by weight of one stereoisomer of the compound and less than about 5% by weight of the other stereoisomers of the compound, or greater than about 97% by weight of one stereoisomer of the compound and less than about 3% by weight of the other stereoisomers of the compound, or greater than about 99% by weight of one stereoisomer of the compound and less than about 1% by weight of the other stereoisomers of the compound. The stereoisomer as described above can be viewed as composition comprising two stereoisomers that are present in their respective weight percentages described herein.

If there is a discrepancy between a depicted structure and a name given to that structure, then the depicted structure controls. Additionally, if the stereochemistry of a structure or a portion of a structure is not indicated with, for example, bold or dashed lines, the structure or portion of the structure is to be interpreted as encompassing all stereoisomers of it. In some cases, however, where more than one chiral center exists, the structures and names may be represented as single enantiomers to help describe the relative stereochemistry. Those skilled in the art of organic synthesis will know if the compounds are prepared as single enantiomers from the methods used to prepare them.

The term "synergy," in various embodiments, can refer to a survival benefit resulting from the combination of Compound 1 and anti-cancer therapy that is greater than the sum of survival benefits from Compound 1 and anti-cancer therapy alone. In some embodiments, synergy is assessed as it inures to the benefit of a particular subject. In other embodiments, synergy is assessed by measuring survival benefits amongst a population of subjects, and then calculating an average or a mean survival benefit of the population. The population of subjects can be chosen by common diagnosis of a particular cancer, such as GBM; stage of cancer; prognosis; and combinations thereof.

In this disclosure, a "pharmaceutically acceptable salt" is a pharmaceutically acceptable, organic or inorganic acid or base salt of a compound described herein. Representative pharmaceutically acceptable salts include, e.g., alkali metal salts, alkali earth salts, ammonium salts, water-soluble and water-insoluble salts, such as the acetate, amsonate (4,4-diaminostilbene-2,2-disulfonate), benzenesulfonate, benzoate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosaliculate, suramate, tannate, tartrate, teoclate, tosylate, triethiodide, and valerate salts. A pharmaceutically acceptable salt can have more than one charged atom in its structure. In this instance the pharmaceutically acceptable salt can have multiple counterions. Thus, a pharmaceutically acceptable salt can have one or more charged atoms and/or one or more counterions.

The terms "treat", "treating" and "treatment" refer to the amelioration or eradication of a disease or symptoms associated with a disease. In various embodiments, the terms refer to minimizing the spread or worsening of the disease resulting from the administration of one or more prophylactic or therapeutic compounds described herein to a patient suffering from such a disease.

The terms "prevent," "preventing," and "prevention" refer to the prevention of the onset, recurrence, or spread of the disease in a patient resulting from the administration of one or more prophylactic or therapeutic compounds described herein.

The term "effective amount" refers to an amount of a compound as described herein or other active ingredient sufficient to provide a therapeutic or prophylactic benefit in the treatment or prevention of a disease or to delay or minimize symptoms associated with a disease. Further, a therapeutically effective amount with respect to a compound as described herein means that amount of therapeutic agent alone, or in combination with other therapies, that provides a therapeutic benefit in the treatment or prevention of a disease. Used in connection with a compound as described herein, the term can encompass an amount that improves overall therapy, reduces or avoids symptoms or causes of disease, or enhances the therapeutic efficacy of or is synergistic with another therapeutic agent.

A "patient" or subject" includes an animal, such as a human, cow, horse, sheep, lamb, pig, cat, dog, mouse, rat, rabbit or guinea pig. In accordance with some embodiments, the animal is a mammal such as a non-primate and a primate (e.g., monkey and human). In one embodiment, a patient is a human, such as a human infant, child, adolescent or adult. In the present disclosure, the terms "patient" and "subject" are used interchangeably.

### METHODS OF USE

In some embodiments, the present disclosure provides a method for the treatment of a cancer in a subject suffering therefrom. The cancer is one that is characterized by rapid tumor proliferation and aggressive invasion of surrounding tissues. In embodiments, the cancer can be defined as one in which NMIIA or NMIIB is expressed at higher levels relative to healthy, i.e., non-cancerous cells, such as those that are adjacent to the cancer. *See, e.g.,* P. Zhou et al., Oncogene 38 (2019) 5500-5515. In some embodiments, levels of NMIIA or NMIIB expression satisfying this criterion generally are elevated, such as about two-fold or greater than that observed in healthy tissue. In other embodiments, regardless of the level of expression of NMIIA or NMIIB, the activity including constitutional activation of NMIIA or NMIIB in the cancer can be increased or decreased, relative to healthy tissue, such as by posttranslational modifications (PTMs) including aberrant phosphorylation and mutations. *See* Nevitt, C. et al., N-terminal acetylation and methylation differentially affect the function of MYL9. Biochem J 2018; 475 (20): 3201-3219.

In various embodiments, cancers that are treatable by the methods described herein include glioblastoma (GBM), pancreatic adenocarcinoma, triple negative breast cancer (TNBC), cholangiocarcinoma, head and neck squamous cell carcinoma, testicular germ cell tumors, liver hepatocellular carcinoma, stomach adenocarcinoma, kidney chromophobe, esophageal carcinoma, brain lower grade glioma, ovarian serous cystadenocarcinoma, thyroid carcinoma, bladder urothelial carcinoma, skin cutaneous melanoma, kidney renal clear cell carcinoma, prostate adenocarcinoma, cervical squamous cell carcinoma, adenocortical carcinoma, lung adenocarcinoma, rectum adenocarcinoma, lung squamous cell carcinoma, breast invasive carcinoma, colon adenocarcinoma, uterine copus endometrial carcinoma, pheochromocytoma and paraganglioma, kidney renal papillary cell carcinoma, uterine carcinosarcoma, acute myeloid leukemia, lymphoid neoplasm diffuse large b-cell lymphoma, mesothelioma, oropharyngeal squamous cell carcinoma, sarcoma, thymoma, and uveal melanoma.

In one embodiment, the cancer is GBM. In another embodiment, the cancer is TNBC. In still another embodiment, the cancer is pancreatic adenocarcinoma. In another embodiment, the cancer is a carcinoma or an adenocarcimona. In another embodiment, the adenocarcinoma is lung adenocarcinoma. In another embodiment, the carcinoma is lung squamous cell carcinoma or breast invasive carcinoma.

The method comprises administering to the subject a combination of Compound 1, or a pharmaceutically acceptable salt thereof: 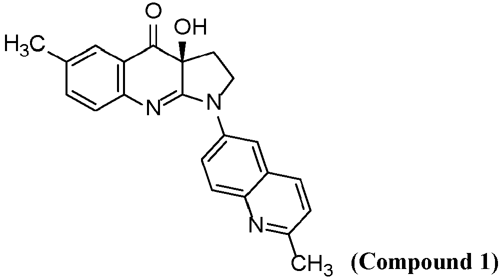 and at least one anti-cancer therapy.

In various embodiments, Compound 1 is administered as a pharmaceutically acceptable salt. In an exemplary embodiment, the pharmaceutically acceptable salt is a mono-hydrochloride salt.

In embodiments, the anti-cancer therapy is selected from:
(a) at least one inhibitor, or a pharmaceutically acceptable salt thereof, of at least one kinase in the RTK-PI3K-mTOR signaling pathway,
(b) at least one inhibitor, or a pharmaceutically acceptable salt thereof, of at least one kinase in the mitogen-activated protein kinase (MAPK) signaling pathway,
(c) an inhibitor of poly (ADP-ribose) polymerase 1 (PARP1), and
(d) a therapeutic dose of radiation.

### Inhibitors of Kinases in RTK-PI3K-mTOR Signaling Pathway

In some embodiments, the anti-cancer therapy is at least one inhibitor, or a pharmaceutically acceptable salt thereof, of at least one kinase in the RTK-PI3K-mTOR signaling pathway. In one embodiment, the kinase in the RTK-PI3K-mTOR signaling pathway is a receptor tyrosine kinase (RTK) and the inhibitor is an RTK inhibitor. In various additional embodiments, Compound 1 is administered in combination with one or more RTK inhibitors generally and specifically disclosed herein.

The RTK inhibitor, in accordance with understanding in the art, refers to a substance, such as a small molecule, that blocks or otherwise inhibits the enzymatic activity of a receptor tyrosine kinase or activities of multiple receptor tyrosine kinases. In various embodiments, the RTK inhibitor inhibits two or more receptor tyrosine kinases, three or more receptor tyrosine kinases, or four or more receptor tyrosine kinases. In some embodiments, more than one RTK inhibitor is administered to the subject. Thus, for example, multiple RTK inhibitors are chosen to inhibit multiple receptor tyrosine kinases. In exemplary embodiments, an RTK inhibitor suitable for use in the methods described herein is one that exhibits at least some brain penetration, CNS permeability, or both.

In various embodiments, the RTK inhibitor is one that targets one or more receptor tyrosine kinases from at least one of the following accepted classes of kinases:
RTK class I (EGF receptor family; ErbB family);
RTK class II (Insulin receptor family);
RTK class III (PDGF and c-KIT receptor family);
RTK class IV (VEGF receptors family);
RTK class V (FGF receptor family);
RTK class VI (CCK receptor family);
RTK class VII (NGF receptor family);
RTK class VIII (HGF receptor family);
RTK class IX (Eph receptor family);
RTK class X (AXL receptor family);
RTK class XI (TIE receptor family);
RTK class XII (RYK receptor family);
RTK class XIII (DDR receptor family);
RTK class XIV (RET receptor family);
RTK class XV (ROS receptor family);
RTK class XVI (LTK receptor family);
RTK class XVII (ROR receptor family);
RTK class XVIII (MuSK receptor family); and
RTK class XIX (LMR receptor).

Many RTK inhibitors are known in the art and are suitable for use in the methods described herein. Thus, Compound 1 in various embodiments is administered in combination with one or more exemplary RTK inhibitors that are selected from fasudil (eril), sirolimus (rapamune), imatinib, gefitinib, erlotinib, sorafenib, sunitinib, saracatinib, dasatinib, lapatinib, nilotinib, temsirolimus, everolimus, pazopanib, ruxolitinib, vandetanib, vemurafenib, crizotinib, icotinib, axitinib, tofacitinib, bosutinib, cabozantinib, ponatinib, regorafenib, afatinib, dabrafenib, trametinib, ibrutinib, nintedanib, idelalisib, alectinib, brigatinib, ceritinib, crizotinib, entrectinib, lorlatinib, bosutinib, dacomitinib, neratinib, osimertinib, gilteritinib, midostaurin, erdafitinib, larotrectinib, axitinib, carbozantinib, lenvatinib, encorafenib, acalabrutinib, binimetinib, cobimetinib, abemaciclib, palbociclib, and ribociclib, and salts and/or hydrates thereof. The RTK inhibitors may inhibit one or more of the RTK classes I through XIX. The selection of RTK inhibitor may be guided by genetic profiling of the glioblastoma. Alternatively, the selection of RTK inhibitor may be guided by anticipated side effects in the patient. In an embodiment, the RTK inhibitor is sunitinib or a salt thereof.

In additional embodiments, the kinase in the RTK-PI3K-mTOR signaling pathway is a phosphoinositide 3-kinase (PI3K) and the inhibitor is a PI3K inhibitor. In various embodiments, Compound 1 is administered in combination with one or more specific examples of PI3K inhibitors suitable for use in the methods described herein including idelalisib, copanlisib, duvelisib, alpelisib, umbralisib, leniolisib, buparlisib, dactolisib, parsaclisib, paxalisib, taselisib, zandelisib, inavolisib, apitolisib, bimiralisib, eganelisib, fimepinostat, gedatolisib, linperlisib, nemiralisib, pictilisib, pilaralisib, samotolisib, seletalisib, serabelisib, sonolisib, tenalisib, voxtalisib, AMG319, AZD8186, GSK2636771, SF1126, LY294002, acalisib, omipalisib, AZD8835, CAL263, GSK1059615, MEN1611, PWT33597, TG100-115, ZSTK474, AEZS-136, B591, GNE-477, hibiscone C, IC87114, PI-103, Wortmannin, pharmaceutically acceptable salts thereof, and combinations thereof. In a particular embodiment, the PI3K inhibitor is paxalisib.

In additional embodiments, the kinase in the RTK-PI3K-mTOR signaling pathway is a mechanistic target of rapamycin (mTOR) kinase and the inhibitor is an mTOR inhibitor. In various embodiments, Compound 1 is administered in combination with one or more specific examples of mTOR inhibitors including rapamycin, temsirolimus, everolimus, ridaforolimus, sirolimus, ridaforolimus, umirolimus, zotarolimus, torin-1, torin-2, vistusertib, pharmaceutically acceptable salts thereof, and combinations thereof. In one embodiment, the mTOR inhibitor is everolimus.

In various embodiments, the anti-cancer therapy is at least one inhibitor or a pharmaceutically acceptable salt thereof of at least one kinase in the MAPK signaling pathway. In some embodiments, the kinase in the MAPK signaling pathway is an Src kinase, and the inhibitor is an Src inhibitor. In various embodiments, Compound 1 is administered in combination with one or more exemplary Src inhibitors including KX2-391, bosutinib, saracatinib, dasatinib, PP1, PP2, pharmaceutically acceptable salts thereof, and combinations thereof. In one embodiment, the Src inhibitor is saracatinib.

In additional embodiments, the kinase in the MAPK signaling pathway is a mitogen-activated protein kinase (MEK), and the inhibitor is a MEK inhibitor. In various embodiments, Compound 1 is administered in combination with one or more specific examples of MEK inhibitors including binimetinib, cobimetinib, selumetinib, trametinib, pharmaceutically acceptable salts thereof, and combinations thereof.

In further embodiments, the kinase in the MAPK signaling pathway is an extracellular signal-regulated kinase (ERK), and the inhibitor is a ERK inhibitor. In various embodiments, Compound 1 is administered in combination with one or more specific examples of ERK inhibitors including ravoxertinib, MK-8353, ulixertinib, temuterkib, KO-947, CC-90003, ONC201, tizaterkib, SCH772984, pluripotin, VX-11e, DEL-22379, FR 180204, ERK5-IN-1, pharmaceutically acceptable salts thereof, and combinations thereof.

In various embodiments, the anti-cancer therapy is a PARP1 inhibitor. In various embodiments, Compound 1 is administered in combination with one or more specific examples of PARP1 inhibitors including rucaparib, iniparib, olaparib, veliparib, niraparib, talazoparib, CEP-9722, and E7016. In an illustrative embodiment, the PARP1 inhibitor is olaparib.

An anti-cancer therapy can be chosen, in various embodiments, in accordance with the established standard of care for a particular cancer. Thus, for example, in embodiments wherein the cancer is pancreatic carcinoma, the anti-cancer therapy can be chosen, in combination with Compound 1, from paclitaxel, fluorouracil (5fu), capecitabine, gemcitabine hydrochloride, irinotecan hydrochloride liposome, mitomycin, everolimus, erlotinib, olaparib, and sunitinib. In additional embodiments, wherein the cancer is pancreatic carcinoma, the anti-cancer therapy in combination with Compound 1 can be chosen from everolimus, sunitinib, and olaparib.

In other embodiments, wherein the cancer is TNBC, the anti-cancer therapy can be chosen, in combination with Compound 1, from fluorouracil (5FU); anthracyclines such as adriamycin; alkylating agents such as cytoxan; taxanes such as taxol and taxotere; paclitaxel, docetaxel, doxorubicin, epirubicin; ADC sacituzumab; PARP inhibitors such as olaparib and talazoparib; growth factor inhibitors such as lapatinib, gefitinib, and cetuximab; mTor inhibitors such as rapamycin; Abl/Src inhibitors such as dasatinib; anti-androgen receptor (AR) therapies such as bicalutamide and enzalutamide; immune checkpoint inhibitors that target PDL1 or PD1, such as atezolizumab and pembrolizumab; and platinum-based chemotherapies such as cisplatin. In additional embodiments, wherein the cancer is TNBC, the anti-cancer therapy in combination with Compound 1 can be chosen from everolimus and olaparib.

In additional embodiments, wherein the cancer is GBM, the anti-cancer therapy can be chosen, in combination with Compound 1, from paxalisib and sunitinib.

In various embodiments, the anti-cancer therapy is a therapeutic dose of radiation. In accordance with established practice in the art, and as used herein, the term "radiation" refers to radiation therapy entailing use of directed X-rays or subatomic particles primarily for cancer management in both curative and palliative settings. In various embodiments, the radiation is administered externally or internally. In one embodiment, the radiation is administered as external beam radiation, wherein a radioactive source outside of the patient emits energy that is focused and shaped to the target of interest. In another embodiment, the radiation resides in brachytherapy, wherein naturally occurring radioactive sources are emplaced, that decay over time, and produce high doses of radiation in a focal area.

In some embodiments, the form of ionizing radiation is the photon. In other embodiments, wherein the radiation dose is needed to increase close to the skin, the form of ionizing radiation is electrons. In still further embodiments, the radiation resides in particles including protons, carbon ions, and neutrons. The skilled person determines, in accordance with sound medical practice, how and where a radiation dose is deposited in the tissue, and knowledge of these patterns can be manipulated to limit the dose to normal structures and thereby improve the therapeutic window.

Impaired DNA repair mechanisms in cancer cells, in contrast to normal cells that rapidly repair double-stranded breaks, renders cancer cells vulnerable to radiation. Therefore, in an embodiment, a total radiation dose can be administered by a fractionated approach, i.e., dividing the total radiation dose over multiple daily treatments: DNA damage in normal cells is repaired between treatments, while damage to cancer cells accumulates over time, causing preferential cancer cell death. Both the dose per fraction and the total dose affect tumor and normal tissue responses. In general, the lower the daily dose of radiation, the less likely it is to cause toxicity. Therefore, in accordance with sound therapeutic practices, the skilled person achieves a balance between daily doses low enough to spare normal tissue but high enough to cause cancer cell death. In exemplary embodiments, a therapeutic dose of radiation can be 180 to 200 cGy per day.

### Administration

The anti-cancer therapy and Compound 1 comprise a combination therapy for treatment of the subject suffering from a cancer described herein. In some embodiments, the anti-cancer therapy and Compound 1 are administered on the same day to the subject. In some embodiments, Compound 1 is administered at one certain time of day, such as the morning, followed by administration of the anti-cancer therapy at another certain time of day, such as the afternoon, or *vice versa.* In other embodiments, Compound 1 and the anti-cancer therapy are administered within a prescribed time interval, such as an hour, of each other. In other embodiments, such as where the anti-cancer therapy is a chemotherapeutic administered through an i.v. route, IP route, or PO route, or subcutaneous route, the anti-cancer therapy is administered simultaneously with Compound 1. In additional embodiments, Compound 1 and the anti-cancer therapy are administered by different routes. For example, in an embodiment, Compound 1 is administered by a subcutaneous route. In other embodiments, the anti-cancer therapy and Compound 1 are administered sequentially, generally where there is a spacing of between 15 to 60 minutes between dosings to monitor for reactions. Additional embodiments reside in more specific combinations of Compound 1 and anti-cancer therapies, in combination with any of the embodiments described herein for dosing, administration of the combination to a subject, and therapeutic endpoints. Thus, in various embodiments the combinations include Compound 1 and tamoxifen, Compound 1 and olaparib, Compound 1 and everolimus, Compound 1 and sunitinib, and Compound 1 and paxalisib.

Numerous dosing regimens provide variations on the combination therapy. For example, in some embodiments, each of the anti-cancer therapy and Compound 1, including the specific combinations thereof as described herein, is administered to the subject once per day on 4 days of each week, on 5 days of each week, or on 6 days of each week. The anti-cancer therapy and Compound 1 can be dosed on the same day, alternating days, and combinations thereof. In some embodiments, the anti-cancer therapy and Compound 1 each is dosed to the subject on each of seven days per week.

Some embodiments define therapeutic endpoints that can vary in accordance with several factors, including age and general health of the subject, presence of co-morbidities, time of cancer diagnosis relative to time that therapeutic intervention begins, and severity of the cancer. In some embodiments, the therapeutic end point is survival of the subject substantially beyond that which would otherwise result from no treatment or from monotherapy with Compound 1 alone or anti-cancer therapy alone. All combinations of Compound 1 and anti-cancer therapy, including those more specifically described herein, are contemplated. In other embodiments, the therapeutic endpoint is subject survival beyond that which would otherwise be expected from the additive effects of monotherapies with Compound 1 and anti-cancer therapy. As used herein, the term "subject" in one embodiment can refer to a specific individual. In another embodiment, the term can refer to a population of individuals for which population a mean or average therapeutic endpoint can determined.

In exemplary embodiments, wherein the cancer is GBM, resection and radiation as treatment results for most patient in an average survival rate of 12-18 months. Those patients with recurrent glioblastoma taking an RTK inhibitor such as sunitinib see little to no additional survival advantage. Thus, in an embodiment, the addition of Compound 1 in accordance with the methods described herein increases the average life expectancy of a patient following resection and radiation to at least 15 months, 18 months, or two years.

In additional embodiments, the therapeutic endpoint of the treatment with the combination is survival of the subject that is about double or greater than double the duration of survival had the subject been administered the anti-cancer therapy or Compound 1 alone. In still other embodiments, the therapeutic endpoint is cancer tumor morbidity, subject survival beyond the time that combination therapy ceases, or both.

Further advantages of the present disclosure arise from the unexpected synergism arising from the combination of Compound 1 and anti-cancer therapy for use in the methods described herein. Thus, in some embodiments, the dosage of the anti-cancer therapy or Compound 1 in the combination is less than the dosage that would be required for the anti-cancer therapy or Compound 1, respectively, if administered alone to the subject to achieve the same therapeutic end point. The ability to exploit lower dosages of Compound 1 or anti-cancer therapy, or both, can minimize or eliminate any toxicity to the subject.

### PHARMACEUTICAL COMPOSITION

The disclosure also provides a pharmaceutical composition comprising a therapeutically effective amount of one or more compounds as described herein, or a pharmaceutically acceptable salt, stereoisomer, and/or tautomer thereof in admixture with a pharmaceutically acceptable carrier. In some embodiments, the composition further contains, in accordance with accepted practices of pharmaceutical compounding, one or more additional therapeutic agents, pharmaceutically acceptable excipients, diluents, adjuvants, stabilizers, emulsifiers, preservatives, colorants, buffers, and/or flavor imparting agents.

The pharmaceutical composition of the present disclosure is formulated, dosed, and administered in a fashion consistent with good medical practice. Factors for consideration in this context include the particular disorder being treated, the particular subject being treated, the clinical condition of the subject, the cause of the disorder, the site of delivery of the agent, the method of administration, the scheduling of administration, and other factors known to medical practitioners.

The "therapeutically effective amount" of a compound or a pharmaceutically acceptable salt, stereoisomer, and/or tautomer thereof that is administered is governed by such considerations. The therapeutically effective amount may be below the amount that is toxic to normal cells, or the subject as a whole. In general, the therapeutically effective amount is the minimum amount necessary for Compound 1 to inhibit NMIIA, inhibit NMIIB, exhibit toxicity to cancer cells, and combinations thereof. In various embodiments, the therapeutically effective amount of Compound 1 is an amount in the range of about 0.1 mg/kg to about 15 mg/kg, about 0.5 mg/kg to about 13 mg/kg, about 1 mg/kg to about 11 mg/kg, or about 5 mg/kg to about 10 mg/kg, based upon weight of the subject. In additional embodiments, the therapeutically effective amount of Compound 1 is about 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, or about 15 mg/kg. Thus, in illustrative embodiments, the combinations include the therapeutically effective amount of Compound 1 as described herein and an anti-cancer therapy (a), anti-cancer therapy (b), anti-cancer therapy (c), or anti-cancer therapy (d). Embodiments include specific combinations of Compound 1 and anti-cancer therapy described herein, including Compound 1 and tamoxifen, Compound 1 and olaparib, Compound 1 and everolimus, Compound 1 and sunitinib, and Compound 1 and paxalisib.

The therapeutically effective amount of an anti-cancer therapy is the minimum amount necessary to exert oncological and/or immuno-oncological therapeutic activity, inhibit one or more receptor tyrosine kinases or other kinases described herein, and combinations thereof. In various embodiments, the therapeutically effective amount of an anti-cancer therapy in combinations with Compound 1 as generally and specifically described herein is an amount in the range of about 0.1 to about 200 mg/kg, about 0.5 to about 150 mg/kg, about 1 to about 125 mg/kg, about 2 to about 110 mg/kg, about 5 mg/kg to about 100 mg/kg, about10 mg/kg to about 80 mg/kg, about 15 mg/kg to about 60 mg/kg, about 20 mg/kg to about 50 mg/kg, or about 30 mg/kg to about 40 mg/kg, based upon weight of the subject. In additional embodiments, the therapeutically effective amount of an anti-cancer therapy is about 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 2 mg/kg, 3 mg/kg, about 4 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, or about 60 mg/kg.

In exemplary embodiments, unit dosage forms include intraperitoneal (IP) injections, intravenous (IV) injections, subcutaneous (SQ) injections, and oral (PO) dosage forms. The dosage forms may contain from about 0.1 mg to about 1000 mg, about 50 mg to about 500 mg, about 25 mg to about 200 mg, or about 10 mg to about 100 mg of Compound 1 or an anti-cancer therapy, or each of Compound 1 or an anti-cancer therapy in admixture with each other. In any of the foregoing embodiments, optionally in combination with any other embodiment described herein, the dosage form can be administered once a day, twice per day, or three times per day.

The compositions of the present disclosure can be administered orally, parenterally, by inhalation or spray or rectally in dosage unit formulations. The term parenteral as used herein includes subcutaneous injections, intravenous, intramuscular, intrasternal injection or infusion techniques.

Suitable oral compositions as described herein include without limitation tablets, troches, lozenges, aqueous or oily suspensions, dispersible powders or granules, emulsion, hard or soft capsules, syrups or elixirs.

In another aspect, also encompassed are pharmaceutical compositions suitable for single unit dosages that comprise a compound of the disclosure or its pharmaceutically acceptable stereoisomer, salt, or tautomer and a pharmaceutically acceptable carrier.

The compositions of the present disclosure that are suitable for oral use may be prepared according to any method known to the art for the manufacture of pharmaceutical compositions. For instance, liquid formulations of the compounds of the present disclosure contain one or more agents selected from the group consisting of sweetening agents, flavoring agents, coloring agents and preserving agents in order to provide pharmaceutically palatable preparations of a compound of the present disclosure.

For tablet compositions, a compound of the present disclosure in admixture with non-toxic pharmaceutically acceptable excipients is used for the manufacture of tablets. Examples of such excipients include without limitation inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known coating techniques to delay disintegration and absorption in the gastrointestinal tract and thereby to provide a sustained therapeutic action over a desired time period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or a medium chain oil.

For aqueous suspensions, a compound of the present disclosure is admixed with excipients suitable for maintaining a stable suspension, such as wherein no compound degradation or precipitation occurs. Examples of such excipients include without limitation are sodium carboxymethylcellulose, methylcellulose, hydroxpropylmethylcellulose, sodium alginate, polyvinylpyrrolidone, gum tragacanth and gum acacia.

Oral suspensions can also contain dispersing or wetting agents, such as naturally-occurring phosphatide, for example, lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending a compound of the present disclosure in a vegetable oil, for example arachis oil, or medium chain oil, or in a mineral oil such as liquid paraffin. The oily suspensions may contain a thickening agent, for example beeswax, hard paraffin or cetyl alcohol.

Sweetening agents such as those set forth above, and flavoring agents may be added to provide palatable oral preparations. These compositions may be preserved by the addition of an anti-oxidant such as ascorbic acid.

Dispersible powders and granules suitable for preparation of an aqueous suspension by the addition of water provide a compound of the present disclosure in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Pharmaceutical compositions of the present disclosure may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monoleate, and condensation reaction products of the said partial esters with ethylene oxide, for example polyoxyethylene sorbitan monoleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative, and flavoring and coloring agents.

The pharmaceutical compositions may be in the form of a sterile injectable, an aqueous suspension or an oleaginous suspension. This suspension may be formulated according to the known art using those suitable dispersing or wetting agents and suspending agents which have been mentioned above. The sterile injectable preparation may also be sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

Compositions for parenteral administrations are administered in a sterile medium. Depending on the vehicle used and concentration the concentration of the drug in the formulation, the parenteral formulation can either be a suspension or a solution containing dissolved drug. Adjuvants such as local anesthetics, preservatives and buffering agents can also be added to parenteral compositions.

### EXAMPLES

The following non-limiting and illustrative examples constitute additional embodiments of the present disclosure.

### Example 1: Combined deletion of NMIIA and IIB profoundly impairs GBM tumorigenesis and prolongs survival in a Genetically Engineered Model (GEMM) of this disease.

A viral-induced mouse model of proneural, IDH wildtype, unmethylated GBM has been described (Lei L, et al., Glioblastoma models reveal the connection between adult glial progenitors and the proneural phenotype. PLoS One. 2011;6(5):e20041). Injection of a bicistronic retrovirus encoding HA-tagged PDGF and Cre recombinase into the white matter of mice with conditional knockouts (cKOs) of a tumor suppressor (Pten or Trp53) led to infection of glial progenitor cells, recombination of the cKO alleles, and malignant transformation with 100% penetrance (Lei L, *et al.* 2011). A comparison of Kaplan Meier survival curves for mice with Pten-deleted GBMs to those with cKOs for Pten, Myh9 (NMIIA) and Myh10 (NMIIB) demonstrated that co-deletion of NMIIA and IIB prevents tumorigenesis and produces a marked improvement in long-term survival.

### Example 2: Compound 1 is a CNS penetrant, well-tolerated dual inhibitor of NMIIA and IIB.

Blebbistatin is an NMII allosteric inhibitor (Straight, A.F., et al., Dissecting temporal and spatial control of cytokinesis with a myosin II Inhibitor. Science, 2003. 299(5613): p. 1743-7) that was shown to be poorly tolerated at doses as low as 0.5 mg/kg (IV) because of its potent inhibition of cardiac myosin II (CMII). In view of the high sequence homology in residues lining the blebbistatin binding site in NMII and CMII, selectively targeting NMII was thought to be intractable. However, Compound 1 exhibits high brain penetrance, strong selectivity profile for NMII over CMII, and importantly, equal potency against both NMIIA and IIB (WO 2019/241469), a feature underlying suppression of both invasion and proliferation. Although Compound 1 exhibits some skeletal (SkMII) and smooth (SmMII) muscle myosin inhibition, safety testing has established CMII as the critical target for toxicity. Additional *in vitro* DMPK data demonstrated that while protein bound, Compound 1 is highly cell permeable and not a P-glycoprotein (Pgp) substrate, in agreement with *in vivo* brain/plasma ratios in mice (blebbistatin (0.9), Compound 1 (2.3) at 10mg/kg). Compound 1 also is non-toxic to healthy cells in culture up to > 30µM.

### Example 3: Combined inhibition of NMIIA and IIB with Compound 1 is cytotoxic to GBM cells in vitro.

Dose-response studies were performed by treating murine and human primary GBM cell lines for 72 h *in vitro* and measuring cell viability. FIG. 1A shows Compound 1 sensitivity of two murine GBM lines: these are a PDGF-driven proneural line that is deleted for Trp53 and a PDGF-driven mesenchymal line (provided by Dr. Hambardzumyan, Mt. Sinai) that is deleted for Nf-1 and suppressed for Trp53, respectively. FIG. 1B shows Compound 1 sensitivity of eight low-passage human GBM lines, three of which have tumor initiating cell (TIC) capability (L0, L1, GBM1A) >Deleyrolle, L.P., et al., Evidence for label-retaining tumour-initiating cells in human glioblastoma. Brain, 2011. 134(Pt 5): p. 1331-43; Tilghman, J., et al., Regulation of Glioblastoma Tumor-Propagating Cells by the Integrin Partner Tetraspanin CD151. Neoplasia, 2016. 18(3): p. 185-98). All tested cell lines were sensitive to Compound 1 with EC₅₀ values in the low micromolar range.

### Example 4. Compound 1 is synergistic with kinase inhibitors in GBM

### General Methods

**Mice.** All mouse procedures were performed in compliance with the Mayo Clinic Institutional Animal Care and Use Committee guidelines. Homozygous floxed Trp53 mice (Stock #008462) were obtained from Jackson Laboratories. Studies were performed on equal numbers of male and female mice, ages 8-20 weeks.

**Glioma cell line isolation from mouse GBM tumor and culture.** The protocol for isolation of tumor cells from Trp53(-/-) murine tumor cells has been described (Lei, L. *et al.* (2011)).

**Dose response curves/cell viability assays.** 5000 cells/well were plated in 96-well plates and were treated 48 hours later with a range of doses of Compound 1, sunitinib, saracatinib, or combinations of 5 µM Compound 1 and a range of concentrations of sunitinib or saracatinib. Cells were treated with drug for 72-96 hours and cell viability was measured using CellTiter-Glo (Promega, cat# G9242). For experiments involving MuSyc analysis to measure drug synergy (Meyer CT, et al., Quantifying Drug Combination Synergy along Potency and Efficacy Axes. Cell Syst., 2019. 8, 97-108) combinations of Compound 1 and sunitinib were added to the 96 well plates and viable cell count was measured with CellTiter-Glo as above. Cell count data was normalized to the signal for vehicle only control.

**Retrovirus production and intracerebral injections.** PDGF-IRES-cre retrovirus was generated and injected intracranially according to methods described previously (Lei, L. *et al.* (2011); Kenchappa RS, et al., Myosin 10 regulates invasion, mitosis, and metabolic signaling in gliobloblastoma. iScience, 2020. Nov 13;23(12):101802). For the pharmacologic studies, mice (with 10 mice per treatment group) were treated five days after retroviral injection with:
- vehicle;
- Compound 1 (10 mg/kg by IP, 7 days per week);
- saracatinib (25 mg/kg by oral gavage, 5 days per week);
- sunitinib (40 mg/kg by oral gavage, 5 days per week);
- Compound 1+sunitinib (10 mg/kg by IP, 7 days per week + 40 mg/kg by oral gavage, 5 days per week); or
- Compound 1+saracatinib (10 mg/kg by IP, 7 days per week + 25 mg/kg by oral gavage, 5 days per week).

Treatment continued until tumor morbidity. Survival data was subjected to Kaplan-Meier analysis and statistical significance of survival differences was assessed with a log rank test (Prizm).

**Drug synergy.** Synergy was calculated using the MuSyC algorithm as previously described (Meyer, CT *et al.* (2019; Wooten DJ, et al., MuSyC is a consensus framework that unifies multi-drug synergy metrics for combinatorial drug discovery. Nat Commun., 2021. 12, 4607). MuSyC quantifies two types of drug synergy, synergistic potency and synergistic efficacy, respectively, both relating to geometric transformations of the dose response surface, which are analogous to the transformations in the 1D Hill equation for potency (horizontal shift in the EC₅₀) and efficacy (vertical shift in Eₘₐₓ). Synergy was calculated by fitting a dose-response surface relating the drug's effect (rate of cell growth or static cell count at 96 hours) to the concentrations of drug 1 and drug 2. To help the growth-rate-based fits converge, the bounds for E0 (effect with no drug) and E1, E2, E3 (maximal effects of drug 1, 2, and the combination) were set to [0.005, 0.002] and [0.01, -0.01], respectively.

### A. In vitro Synergism with RTK Inhibitors

This example examined the effects of 5 µM Compound 1 on the dose response of Trp53-deleted (Trp53(-/-)) murine GBM cells to two FDA-approved, CNS permeant therapeutics, saracantinib, which is a combined SRC/EGFR inhibitor, and sunitinib, which is a PDGFR, VEGFR, and c-KIT inhibitor. Compound 1 shifted the EC₅₀ for sunitinib to the left by ~20-fold (FIG. 4A), and for saracatinib by >1000-fold (FIG. 4B). Taken together, these *in vitro* results demonstrate a synergism in coadministration of Compound 1 and RTK inhibitor in preclinical models of GBM.

Synergy *in vitro* between sunitinib and Compound 1 was analyzed with MuSyC, which fits a dose-response surface to drug combination data to calculate the degree of synergistic efficacy (β) and synergistic potency (log(α12) and log(α21)). Unlike isobologram or combined index analysis, MuSyC can derive synergies of potency and efficacy. Fig. 2A illustrates a MuSyC dose response surface for the combination of sunitinib and Compound 1, showing that efficacy is increased by this combination by 42% compared to either drug alone (β=0.42), and that the combination increases sunitinib's potency, reducing its EC₅₀ >8.5-fold in the presence of Compound 1 (Fig. 2B).

### B. In vivo Synergism with RTK Inhibitors and GBM Survival

In this example, a Trp53-deleted genetically engineered model (GEMM) of GBM was utilized to test the therapeutic benefit of Compound 1, as this model is uniformly lethal by 35 days post-retroviral injection. Administration of Compound 1 began on day 5 post-retroviral injection and stopped at tumor morbidity. The Kaplan Meier survival curves for these mice showed that single agent Compound 1 increased median survival over vehicle treatment by 32% in this group (FIG. 3; p< 0.0001, log rank test).

To confirm the *in vitro* synergism between Compound 1 and RTK inhibitors, Compound 1 was co-administered with sunitinib *in vivo* in the Trp53 (-/-) GEMM. Like Compound 1, single agent sunitinib enhanced survival over vehicle by ~32%. However, the combination of both drugs markedly enhanced survival beyond either drug alone, i.e., >50% the sum of each drug alone (FIG. 3).

In this example, which represents two combined experiments for replication purposes, mice were treated until they developed tumor morbidity or until day 85 post-retroviral injection, whichever came first. Median survival with the synergistic combination doubled compared to either drug alone. Furthermore, ~35% of mice remained alive at day 100, 15 days after cessation of active therapy. Necropsy of these mice revealed no evidence of tumor, as measured immunohistochemically using the HA epitope to observe PDGF-secreting cells. The results therefore demonstrate surprising long-term survival, especially in this highly aggressive GBM model.

### C. In vivo Synergism with PI3K Inhibitors and GBM Survival

Following the procedures described above, P53-deleted GBMs were induced in mice with homozygous conditional knockouts of p53 by orthotopic injection of the PDGF-IRES-cre retrovirus. Seven days later, animals were injected daily with vehicle, Compound 1, paxalisib, or a combination of Compound 1 and paxalisib and followed for survival. The results show that the combination of Compound 1 and paxalisib effected a surprising increase in mouse survival in comparison to mice treated with Compound 1 or paxalisib alone **(****FIG. 5****).**

### Example 5. Compound 1 is synergistic with radiation

The purpose of this example is to show that Compound 1 sensitizes GBM to radiation in a synergistic manner. The combination of Compound 1 (5 mg/kg, SC) with radiation (2 Gy/dose x 5 daily doses) prolonged mouse survival to a degree greater than the sum of each treatment in both a human patient-derived xenograft (PDX) model **(****FIG. 6A****)** and a p53-deleted genetically engineered mouse model (GEMM) **(****FIG. 6B****).**

### Example 6. Compound 1 is synergistic with kinase inhibitors in Pancreatic Cancer and TNBC

**General Methods.** Cancer cells were seeded in a 96-well culture plate at a density of 1000-2000 cells per well. After a 24-hour incubation period, the cells were treated with the specified compounds, maintaining either a constant or non-constant ratio of concentration between the two compounds. Cell viability was assessed using the Cell Titer-Glo^{®} Luminescent Cell Viability Assay from Promega. The resulting data were analyzed and visualized using GraphPad Prism 10. To quantify synergistic effects, the Combination Index (CI) was calculated using the CompuSyn computer software. A CI value below 1 indicates synergism, with lower CI values reflecting stronger synergistic effects. Further details and analysis tools can be found at https://www.combosyn.com/.

### A. Pancreatic Cancer

This example demonstrates the synergistic effect of Compound 1 in combination with three inhibitors (everolimus, sunitinib, and olaparib) in pancreatic carcinoma cell lines. The cells were subjected to treatment with Compound 1 alone, inhibitors alone, and a combination of Compound 1 with each inhibitor, maintaining a specified constant concentration ratio as indicated in the accompanying figures over a 5-day duration. The evaluation of synergistic effects was conducted using the CompuSyn computer software as described herein.

**Everolimus/PANC-1.** For Compound 1 and everolimus in PANC-1, a Combination Index (CI) below 1 was achieved within a Fractional Viability range of 0.3 to 0.85 (FIG. 7A and FIG. 7B).

**Sunitinib/PANC-1.** For Compound 1 and sunitinib in PANC-1, a CI below 1 was achieved when the Fractional Viability was less than 0.3 (FIG. 8A and FIG. 8B).

**Olaparib/PANC-1.** For Compound 1 and olaparib in PANC-1, a CI below 1 was achieved when the Fractional Viability exceeded 0.3 (FIG. 9A and FIG. 9B).

**Sunitinib/MIA PaCa-2.** For Compound 1 and sunitinib in MIA PaCa-2, a CI below 1 was achieved when the Fractional Viability was less than 0.2 (FIG. 10A and FIG. 10B).

**Olaparib /MIA PaCa-2.** For Compound 1 and olaparib in MIA PaCa-2, a CI below 1 was achieved when the Fractional Viability was less than 0.7 (FIG. 11A and FIG. 11B).

### B. Triple Negative Breast Cancer (TNBC)

This example demonstrates the synergistic effect of Compound 1 in combination with everolimus against a TNBC cell line (MDA-MB-468). The cells were subjected to treatment with Compound 1 alone, everolimus alone, and a combination of Compound 1 (5 µM) with everolimus. The combination utilized a non-constant ratio of concentration and was administered for 5 days. The evaluation of synergistic effects was conducted using the CompuSyn computer software as described herein. Compound 1 and everolimus in MDA-MB-468 demonstrated a Combination Index (CI) below 1 across the entire tested range, with Fractional Viability ranging from 0.52 to 0.38 (FIG. 11A and FIG. 11B).

### Example 7. Synergy of Compound 1 with FDA-approved Drugs

This example presents additional data demonstrating synergistic combinations of Compound 1 with additional anti-cancer therapy drugs against various tumor cell lines.

The cells indicated in Table 2 below underwent treatment with Compound 1 alone, anti-cancer therapy drugs alone, and a combination of Compound 1 with each drug. Compound 1 was maintained at a concentration of 5 µM, with a non-constant ratio of the two compounds or with a specified constant concentration ratio over a 2, 3, or 5-day duration. To assess synergistic effects, the Combination Index (CI) was calculated using the following formula: $CI = \left(D\right) 1 / \left(Dx\right) 1 + \left(D\right) 2 / \left(Dx\right) 2 + \left(D\right) 1 \left(D\right) 2 / \left(Dx\right) 1 \left(Dx\right) 2$

Here, (D)1 and (D)2 represent the doses of Compound 1 and drug, respectively, in the combination that produce 50% efficacy (Fa=0.5), while (Dx)1 and (Dx)2 represent the doses of Compound 1 and the tested drug individually that produce 50% efficacy. A CI value below 1 indicates synergism, with lower CI values reflecting stronger synergistic effects.

**Table 2**

| Type of cancer | Cell lines | Time of Incubation (days) | Drug | EC₅₀ of compound 1 (µM) | EC₅₀ of drug (µM) | Compound 1 (µM) at Fa=0.5 in combination | drug (µM) at Fa=0.5 in combination | Combination Index (CI) at Fa=0.5 |
|---|---|---|---|---|---|---|---|---|
| Breast cancer | MCF-7 | 5 | Tamoxifen | 15.3 | 4 | 5 | 0.61 | 0.53 |
| | | 3 | Olaparib | 9.4 | 197 | 5 | 0.32 | 0.53 |
| | | 2 | Everolimus | 9.4 | 0.6 | 5 | 0.01 | 0.56 |
| | T47D | 5 | Tamoxifen | 6.5 | 19.59 | 5 | 0.1437 | 0.78 |
| | | 3 | Olaparib | 7.5 | 14.2 | 5 | 0.055 | 0.67 |
| | | 2 | Everolimus | 7.5 | 15.5 | 5 | 0.06 | 0.67 |
| | MDA-MB-468 | 5 | Everolimus | 10.16 | 10 | 5 | 0.05 | 0.50 |
| Pancreatic adenocarcinoma | MIA PaCa-2 | 5 | Olaparib | 6 | 7 | 0.94 | 4.41 | 0.89 |
| | | 5 | Everolimus | 6 | 6.4 | 5 | 0.02 | 0.84 |
| | PANC-1 | 5 | Everolimus | 25.1 | 102.7 | 16.7 | 10.2 | 0.83 |

## Claims

1. Compound 1, or a pharmaceutically acceptable salt thereof, in combination with at least one anti-cancer therapy for use in the treatment of cancer in a subject suffering therefrom, wherein the cancer is **characterized by** rapid tumor cell proliferation and aggressive invasion of surrounding tissues, wherein Compound 1 has the structure:

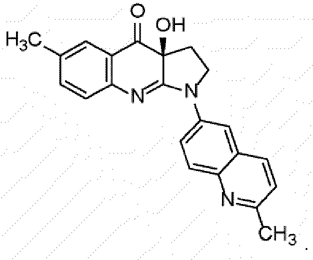

2. Compound 1, or the pharmaceutically acceptable salt thereof, in combination with at least one anti-cancer therapy for use according to claim 1, wherein the cancer is selected from the group consisting of glioblastoma (GBM), pancreatic adenocarcinoma, triple negative breast cancer (TNBC), cholangiocarcinoma, head and neck squamous cell carcinoma, testicular germ cell tumors, liver hepatocellular carcinoma, stomach adenocarcinoma, kidney chromophobe, esophageal carcinoma, brain lower grade glioma, ovarian serous cystadenocarcinoma, thyroid carcinoma, bladder urothelial carcinoma, skin cutaneous melanoma, kidney renal clear cell carcinoma, prostate adenocarcinoma, cervical squamous cell carcinoma, adenocortical carcinoma, lung adenocarcinoma, rectum adenocarcinoma, lung squamous cell carcinoma, breast invasive carcinoma, colon adenocarcinoma, uterine copus endometrial carcinoma, pheochromocytoma and paraganglioma, kidney renal papillary cell carcinoma, uterine carcinosarcoma, acute myeloid leukemia, lymphoid neoplasm diffuse large b-cell lymphoma, mesothelioma, oropharyngeal squamous cell carcinoma, sarcoma, thymoma, and uveal melanoma, preferably wherein the cancer is GBM, TNBC, or pancreatic adenocarcinoma.

3. Compound 1, or the pharmaceutically acceptable salt thereof, in combination with at least one anti-cancer therapy for use according to claim 1 or 2, wherein Compound 1 is the mono-hydrochloride salt of Compound 1.

4. Compound 1, or the pharmaceutically acceptable salt thereof, in combination with at least one anti-cancer therapy for use according to any one of claims 1-3, wherein the anti-cancer therapy is a receptor tyrosine kinase (RTK) inhibitor selected from the group consisting of fasudil (eril), sirolimus (rapamune), imatinib, gefitinib, erlotinib, sorafenib, sunitinib, saracatinib, dasatinib, lapatinib, nilotinib, temsirolimus, everolimus, pazopanib, ruxolitinib, vandetanib, vemurafenib, crizotinib, icotinib, axitinib, tofacitinib, bosutinib, cabozantinib, ponatinib, regorafenib, afatinib, dabrafenib, trametinib, ibrutinib, nintedanib, idelalisib, alectinib, brigatinib, ceritinib, crizotinib, entrectinib, lorlatinib, bosutinib, dacomitinib, neratinib, osimertinib, gilteritinib, midostaurin, erdafitinib, larotrectinib, axitinib, carbozantinib, lenvatinib, regorafenib, dabrafenib, encorafenib, acalabrutinib, binimetinib, cobimetinib, abemaciclib, palbociclib, ribociclib, pharmaceutically acceptable salts thereof, and combinations thereof, preferably wherein the RTK inhibitor is sunitinib.

5. Compound 1, or the pharmaceutically acceptable salt thereof, in combination with at least one anti-cancer therapy for use according to any one of claims 1-3, wherein the anti-cancer therapy is a PI3K inhibitor selected from the group consisting of idelalisib, copanlisib, duvelisib, alpelisib, umbralisib, leniolisib, buparlisib, dactolisib, parsaclisib, paxalisib, taselisib, zandelisib, inavolisib, apitolisib, bimiralisib, eganelisib, fimepinostat, gedatolisib, linperlisib, nemiralisib, pictilisib, pilaralisib, samotolisib, seletalisib, serabelisib, sonolisib, tenalisib, voxtalisib, AMG319, AZD8186, GSK2636771, SF1126, LY294002, acalisib, omipalisib, AZD8835, CAL263, GSK1059615, MEN1611, PWT33597, TG100-115, ZSTK474, AEZS-136, B591, GNE-477, hibiscone C, IC87114, PI-103, wortmannin, pharmaceutically acceptable salts thereof, and combinations thereof, preferably wherein the PI3K inhibitor is paxalisib.

6. Compound 1, or the pharmaceutically acceptable salt thereof, in combination with at least one anti-cancer therapy for use according to any one of claims 1-3, wherein the anti-cancer therapy is an mTOR inhibitor selected from the group consisting of rapamycin, temsirolimus, everolimus, ridaforolimus, sirolimus, ridaforolimus, umirolimus, zotarolimus, torin-1, torin-2, vistusertib, pharmaceutically acceptable salts thereof, and combinations thereof, preferably wherein the mTOR inhibitor is everolimus.

7. Compound 1, or the pharmaceutically acceptable salt thereof, in combination with at least one anti-cancer therapy for use according to any one of claims 1-3, wherein the anticancer therapy is a Src inhibitor selected from the group consisting of KX2-391, bosutinib, saracatinib, dasatinib, PPI, PP2, pharmaceutically acceptable salts thereof, and combinations thereof, preferably wherein the Src inhibitor is saracatinib.

8. Compound 1, or the pharmaceutically acceptable salt thereof, in combination with at least one anti-cancer therapy for use according to any one of claims 1-3, wherein the anti-cancer therapy is a MEK inhibitor selected from the group consisting of binimetinib, cobimetinib, selumetinib, trametinib, pharmaceutically acceptable salts thereof, and combinations thereof.

9. Compound 1, or the pharmaceutically acceptable salt thereof, in combination with at least one anti-cancer therapy for use according to any one of claims 1-3, wherein the anti-cancer therapy is an ERK inhibitor selected from the group consisting of ravoxertinib, MK-8353, ulixertinib, temuterkib, KO-947, CC-90003, ONC201, tizaterkib, SCH772984, pluripotin, VX-l le, DEL-22379, FR 180204, ERK5-IN-1, pharmaceutically acceptable salts thereof, and combinations thereof.

10. Compound 1, or the pharmaceutically acceptable salt thereof, in combination with at least one anti-cancer therapy for use according to any one of claims 1-3, wherein the anti-cancer therapy is a PARP1 inhibitor selected from the group consisting of rucaparib, iniparib, olaparib, veliparib, niraparib, talazoparib, CEP-9722, and E7016, preferably wherein the PARP1 inhibitor is olaparib.

11. Compound 1, or the pharmaceutically acceptable salt thereof, in combination with at least one anti-cancer therapy for use according to any one of claims 1-3, wherein the anti-cancer therapy is tamoxifen.

12. Compound 1, or the pharmaceutically acceptable salt thereof, in combination with at least one anti-cancer therapy for use according to any one of claims 1-10, wherein Compound 1 and the at least one anti-cancer therapy are administered to the subject once per day on 4 days of each week, once per day on 5 days of each week, once per day on 6 days of each week, or once per day on 7 days of each week.

13. Compound 1, or the pharmaceutically acceptable salt thereof, in combination with at least one anti-cancer therapy for use according to any one of claims 1-12, wherein the dosage of the anti-cancer therapy or Compound 1 is less than the dosage that would be required for the anti-cancer therapy or Compound 1 if either were to be administered alone to the subject to achieve the same therapeutic endpoint.

14. Compound 1, or the pharmaceutically acceptable salt thereof, in combination with at least one anti-cancer therapy for use according to any one of claims 1-13, wherein the therapeutic endpoint of administering Compound 1 in combination with at least one anti-cancer therapy is survival of the subject longer than survival had the subject been administered the anti-cancer agent or Compound 1 alone.

15. Compound 1, or the pharmaceutically acceptable salt thereof, in combination with at least one anti-cancer therapy for use according to any one of claims 1-14, wherein each dose of Compound 1 in the combination is about 0.1 to about 15 mg/kg.

## Patentansprüche

1. Verbindung 1, oder ein pharmazeutisch verträgliches Salz davon, in Kombination mit mindestens einer Antikrebstherapie zur Verwendung bei der Behandlung von Krebs bei einem daran leidenden Subjekt, wobei der Krebs durch schnelle Tumorzellproliferation und aggressive Invasion von umgebenden Geweben gekennzeichnet ist, wobei Verbindung 1 die folgende Struktur aufweist:

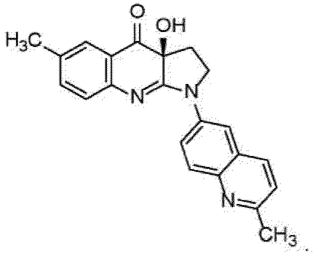

2. Verbindung 1, oder das pharmazeutisch verträgliche Salz davon, in Kombination mit mindestens einer Antikrebstherapie zur Verwendung nach Anspruch 1, wobei der Krebs ausgewählt ist aus der Gruppe bestehend aus Glioblastom (GBM), Pankreasadenokarzinom, dreifach negativem Brustkrebs (TNBC), Cholangiokarzinom, Kopf- und Hals-Plattenepithelkarzinom, Hodenkeimzelltumoren, hepatozellulärem Leberkarzinom, Magenadenokarzinom, Nieren-Chromophobe, Ösophaguskarzinom, Hirngliom niedrigeren Grades, serösem Eierstock-Zystadenokarzinom, Schilddrüsenkarzinom, Blasen-Urothelkarzinom, kutanem Melanom der Haut, renalem Reinzellkarzinom der Niere, Prostatadenokarzinom, Plattenzellkarzinom des Gebärmutterhalses, adenokortikalem Karzinom, Lungenadenokarzinom, Rektumadenokarzinom, Plattenzellkarzinom der Lunge, brustinvasivem Karzinom, Kolon-Adenokarzinom, Uterus-corpus-Endometriumkarzinom, Pheochromozytom und Paragangliom, Papillärzellenkarzinom der Niere, Uterus-Karzinosarkom, akuter myeloider Leukämie, lymphoidem Neoplasma, diffusem großzelligem B-Zell-Lymphom, Mesotheliom, oropharyngealem Plattenepithelkarzinom, Sarkom, Thymom und Aderhautmelanom, bevorzugt, wobei der Krebs GBM, TNBC oder Pankreasadenokarzinom ist.

3. Verbindung 1, oder das pharmazeutisch verträgliche Salz davon, in Kombination mit mindestens einer Antikrebstherapie zur Verwendung nach Anspruch 1 oder 2, wobei Verbindung 1 das Monohydrochloridsalz von Verbindung 1 ist.

4. Verbindung 1, oder das pharmazeutisch verträgliche Salz davon, in Kombination mit mindestens einer Antikrebstherapie zur Verwendung nach einem der Ansprüche 1-3, wobei die Antikrebstherapie ein Rezeptortyrosinkinaseinhibitor(RTK)-Inhibitor ist, der ausgewählt ist aus der Gruppe bestehend aus Fasudil (Eril), Sirolimus (Rapamune), Imatinib, Gefitinib, Erlotinib, Sorafenib, Sunitinib, Saracatinib, Dasatinib, Lapatinib, Nilotinib, Temsirolimus, Everolimus, Pazopanib, Ruxolitinib, Vandetanib, Vemurafenib, Crizotinib, Icotinib, Axitinib, Tofacitinib, Bosutinib, Cabozantinib, Ponatinib, Regorafenib, Afatinib, Dabrafenib, Trametinib, Ibrutinib, Nintedanib, Idelalisib, Alectinib, Brigatinib, Ceritinib, Crizotinib, Entrectinib, Lorlatinib, Bosutinib, Dacomitinib, Neratinib, Osimertinib, Gilteritinib, Midostaurin, Erdafitinib, Larotrectinib, Axitinib, Carbozantinib, Lenvatinib, Regorafenib, Dabrafenib, Encorafenib, Acalabrutinib, Binimetinib, Cobimetinib, Abemaciclib, Palbociclib, Ribociclib, pharmazeutisch verträglichen Salzen davon und Kombinationen davon, bevorzugt, wobei der RTK-Inhibitor Sunitinib ist.

5. Verbindung 1, oder das pharmazeutisch verträgliche Salz davon, in Kombination mit mindestens einer Antikrebstherapie zur Verwendung nach einem der Ansprüche 1-3, wobei die Antikrebstherapie ein PI3K-Inhibitor ist, der ausgewählt ist aus der Gruppe bestehend aus Idelalisib, Copanlisib, Duvelisib, Alpelisib, Umbralisib, Leniolisib, Buparlisib, Dactolisib, Parsaclisib, Paxalisib, Taselisib, Zandelisib, Inavolisib, Apitolisib, Bimiralisib, Eganelisib, Fimepinostat, Gedatolisib, Linperlisib, Nemiralisib, Pictilisib, Pilaralisib, Samotolisib, Seletalisib, Serabelisib, Sonolisib, Tenalisib, Voxtalisib, AMG319, AZD8186, GSK2636771, SF1126, LY294002, Acalisib, Omipalisib, AZD8835, CAL263, GSK1059615, MEN1611, PWT33597, TG100-115, ZSTK474, AEZS-136, B591, GNE-477, Hibiscon C, IC87114, PI-103, Wortmannin, pharmazeutisch verträglichen Salzen davon und Kombinationen davon, bevorzugt, wobei der PI3K-Inhibitor Paxalisib ist.

6. Verbindung 1, oder das pharmazeutisch verträgliche Salz davon, in Kombination mit mindestens einer Antikrebstherapie zur Verwendung nach einem der Ansprüche 1-3, wobei die Antikrebstherapie ein mTOR-Inhibitor ist, der ausgewählt ist aus der Gruppe bestehend aus Rapamycin, Temsirolimus, Everolimus, Ridaforolimus, Sirolimus, Ridaforolimus, Umirolimus, Zotarolimus, Torin-1, Torin-2, Vistusertib, pharmazeutisch verträglichen Salzen davon und Kombinationen davon, bevorzugt, wobei der mTOR-Inhibitor Everolimus ist.

7. Verbindung 1, oder das pharmazeutisch verträgliche Salz davon, in Kombination mit mindestens einer Antikrebstherapie zur Verwendung nach einem der Ansprüche 1-3, wobei die Antikrebstherapie ein Src-Inhibitor ist, der ausgewählt ist aus der Gruppe bestehend aus KX2-391, Bosutinib, Saracatinib, Dasatinib, PPI, PP2, pharmazeutisch verträglichen Salzen davon und Kombinationen davon, bevorzugt, wobei der Src-Inhibitor Saracatinib ist.

8. Verbindung 1, oder das pharmazeutisch verträgliche Salz davon, in Kombination mit mindestens einer Antikrebstherapie zur Verwendung nach einem der Ansprüche 1-3, wobei die Antikrebstherapie ein MEK-Inhibitor ist, der ausgewählt ist aus der Gruppe bestehend aus Binimetinib, Cobimetinib, Selumetinib, Trametinib, pharmazeutisch verträglichen Salzen davon und Kombinationen davon.

9. Verbindung 1, oder das pharmazeutisch verträgliche Salz davon, in Kombination mit mindestens einer Antikrebstherapie zur Verwendung nach einem der Ansprüche 1-3, wobei die Antikrebstherapie ein ERK-Inhibitor ist, der ausgewählt ist aus der Gruppe bestehend aus Ravoxertinib, MK-8353, Ulixertinib, Temuterkib, KO-947, CC-90003, ONC201, Tizaterkib, SCH772984, Pluripotin, VX-1 le, DEL-22379, FR 180204, ERK5-IN-1, pharmazeutisch verträglichen Salzen davon und Kombinationen davon.

10. Verbindung 1, oder das pharmazeutisch verträgliche Salz davon, in Kombination mit mindestens einer Antikrebstherapie zur Verwendung nach einem der Ansprüche 1-3, wobei die Antikrebstherapie ein PARP1-Inhibitor ist, der ausgewählt ist aus der Gruppe bestehend aus Rucaparib, Iniparib, Olaparib, Veliparib, Niraparib, Talazoparib, CEP-9722 und E7016, bevorzugt, wobei der PARP1-Inhibitor Olaparib ist.

11. Verbindung 1, oder das pharmazeutisch verträgliche Salz davon, in Kombination mit mindestens einer Antikrebstherapie zur Verwendung nach einem der Ansprüche 1-3, wobei die Antikrebstherapie Tamoxifen ist.

12. Verbindung 1, oder das pharmazeutisch verträgliche Salz davon, in Kombination mit mindestens einer Antikrebstherapie zur Verwendung nach einem der Ansprüche 1-10, wobei Verbindung 1 und die mindestens eine Antikrebstherapie dem Subjekt einmal pro Tag an 4 Tagen jeder Woche, einmal pro Tag an 5 Tagen jeder Woche, einmal pro Tag an 6 Tagen jeder Woche oder einmal pro Tag an 7 Tagen jeder Woche verabreicht werden.

13. Verbindung 1, oder das pharmazeutisch verträgliche Salz davon, in Kombination mit mindestens einer Antikrebstherapie zur Verwendung nach einem der Ansprüche 1-12, wobei die Dosierung der Antikrebstherapie oder Verbindung 1 weniger ist als die Dosierung, die für die Antikrebstherapie oder Verbindung 1 erforderlich wäre, wenn eine von beiden dem Subjekt allein verabreicht würde, um den gleichen therapeutischen Endpunkt zu erreichen.

14. Verbindung 1, oder das pharmazeutisch verträgliche Salz davon, in Kombination mit mindestens einer Antikrebstherapie zur Verwendung nach einem der Ansprüche 1-13, wobei der therapeutische Endpunkt der Verabreichung von Verbindung 1 in Kombination mit mindestens einer Antikrebstherapie das Überleben des Subjekts länger als das Überleben ist, wenn dem Subjekt das Antikrebsmittel oder Verbindung 1 allein verabreicht worden wäre.

15. Verbindung 1, oder das pharmazeutisch verträgliche Salz davon, in Kombination mit mindestens einer Antikrebstherapie zur Verwendung nach einem der Ansprüche 1-14, wobei jede Dosis von Verbindung 1 in der Kombination etwa 0,1 bis etwa 15 mg/kg beträgt.

## Revendications

1. Composé 1, ou sel acceptable pharmaceutiquement de celui-ci, en combinaison avec au moins une thérapie anticancéreuse, pour une utilisation dans le traitement d'un cancer chez un sujet qui en est atteint, dans lequel le cancer est **caractérisé par** une prolifération rapide de cellules tumorales et une invasion agressive de tissus environnants, dans lequel le Composé 1 répond à la structure :

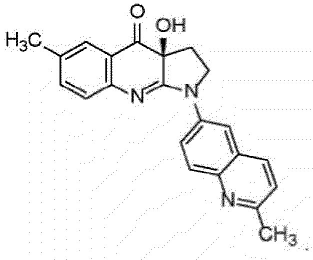

2. Composé 1, ou sel acceptable pharmaceutiquement de celui-ci, en combinaison avec au moins une thérapie anticancéreuse, pour une utilisation selon la revendication 1, dans lequel le cancer est choisi dans le groupe constitué du glioblastome (GBM), de l'adénocarcinome pancréatique, du cancer du sein triple négatif (TNBC), du cholangiocarcinome, du carcinome épidermoïde de la tête et du cou, des tumeurs germinales testiculaires, du carcinome hépatocellulaire du foie, de l'adénocarcinome de l'estomac, du carcinome/cancer chromophobe du rein, du carcinome de l'œsophage, du gliome cérébral de bas grade, du cystadénocarcinome séreux de l'ovaire, du carcinome de la thyroïde, du carcinome urothélial de la vessie, du mélanome cutané, du carcinome à cellules claires du rein, de l'adénocarcinome de la prostate, du carcinome épidermoïde du col de l'utérus, du carcinome corticosurrénalien, de l'adénocarcinome du poumon, de l'adénocarcinome du rectum, du carcinome épidermoïde du poumon, du carcinome invasif du sein, de l'adénocarcinome du côlon, du carcinome endométrial du corps utérin, du phéochromocytome et du paragangliome, du carcinome papillaire rénal, du carcinosarcome utérin, de la leucémie myéloïde aiguë, du néoplasme lymphoïde lymphome diffus à grandes cellules B, du mésothéliome, du carcinome épidermoïde de l'oropharynx, du sarcome, du thymome et du mélanome uvéal, de préférence dans lequel le cancer est le GBM, le TNBC ou l'adénocarcinome pancréatique.

3. Composé 1, ou sel acceptable pharmaceutiquement de celui-ci, en combinaison avec au moins une thérapie anticancéreuse, pour une utilisation selon l'une des revendications 1 ou 2, dans lequel le Composé 1 est le sel monochlorhydrate du Composé 1.

4. Composé 1, ou sel acceptable pharmaceutiquement de celui-ci, en combinaison avec au moins une thérapie anticancéreuse, pour une utilisation selon l'une quelconque des revendications 1 à 3, dans lequel la thérapie anticancéreuse est un inhibiteur de récepteur tyrosine kinase (RTK) choisi dans le groupe constitué du fasudil (eril), du sirolimus (rapamune), de l'imatinib, du géfitinib, de l'erlotinib, du sorafénib, du sunitinib, du saracatinib, du dasatinib, du lapatinib, du nilotinib, du temsirolimus, de l'évérolimus, du pazopanib, du ruxolitinib, du vandétanib, du vémurafénib, du crizotinib, de l'icotinib, de l'axitinib, du tofacitinib, du bosutinib, du cabozantinib, du ponatinib, du régorafénib, de l'afatinib, du dabrafénib, du tramétinib, de l'ibrutinib, du nintédanib, de l'idélalisib, de l'alectinib, du brigatinib, du céritinib, du crizotinib, de l'entrectinib, du lorlatinib, du bosutinib, du dacomitinib, du nératinib, de l'osimertinib, du giltéritinib, de la midostaurine, de l'erdafitinib, du larotrectinib, de l'axitinib, du carbozantinib, du lenvatinib, du régorafénib, du dabrafénib, de l'encorafénib, de l'acalabrutinib, du binimétinib, du cobimétinib, de l'abémaciclib, du palbociclib, du ribociclib, de sels acceptables pharmaceutiquement de ceux-ci, et de combinaisons de ceux-ci, de préférence dans lequel l'inhibiteur de RTK est le sunitinib.

5. Composé 1, ou sel acceptable pharmaceutiquement de celui-ci, en combinaison avec au moins une thérapie anticancéreuse, pour une utilisation selon l'une quelconque des revendications 1 à 3, dans lequel la thérapie anticancéreuse est un inhibiteur de PI3K choisi dans le groupe constitué de l'idélalisib, du copanlisib, du duvélisib, de l'alpélisib, de l'umbralisib, du léniolisib, du buparlisib, du dactolisib, du parsaclisib, du paxalisib, du tasélisib, du zandélisib, de l'inavolisib, de l'apitolisib, du bimiralisib, de l'éganélisib, du fimepinostat, du gedatolisib, du linpérlisib, du némiralisib, du pictilisib, du pilaralisib, du samotolisib, du séletalisib, du sérabélisib, du sonolisib, du ténalisib, du voxtalisib, de l'AMG319, de l'AZD8186, du GSK2636771, du SF1126, du LY294002, de l'acalisib, de l'omipalisib, de l'AZD8835, du CAL263, du GSK1059615, du MEN1611, du PWT33597, du TG100-115, du ZSTK474, de l'AEZS-136, du B591, du GNE-477, de l'hibiscone C, de l'IC87114, du PI-103, de la wortmannine, de sels acceptables pharmaceutiquement de ceux-ci, et de combinaisons de ceux-ci, de préférence dans lequel l'inhibiteur de PI3K est le paxalisib.

6. Composé 1, ou sel acceptable pharmaceutiquement de celui-ci, en combinaison avec au moins une thérapie anticancéreuse, pour une utilisation selon l'une quelconque des revendications 1 à 3, dans lequel la thérapie anticancéreuse est un inhibiteur de mTOR choisi dans le groupe constitué de la rapamycine, du temsirolimus, de l'évérolimus, du ridaforolimus, du sirolimus, du ridaforolimus, de l'umirolimus, du zotarolimus, du torin-1, du torin-2, du vistusertib, de sels acceptables pharmaceutiquement de ceux-ci et de combinaisons de ceux-ci, de préférence dans lequel l'inhibiteur de mTOR est l'évérolimus.

7. Composé 1, ou sel acceptable pharmaceutiquement de celui-ci, en combinaison avec au moins une thérapie anticancéreuse, pour une utilisation selon l'une quelconque des revendications 1 à 3, dans lequel la thérapie anticancéreuse est un inhibiteur de Src choisi dans le groupe constitué du KX2-391, du bosutinib, du saracatinib, du dasatinib, du PPI, du PP2, de sels acceptables pharmaceutiquement de ceux-ci, et de combinaisons de ceux-ci, de préférence dans lequel l'inhibiteur de Src est le saracatinib.

8. Composé 1, ou sel acceptable pharmaceutiquement de celui-ci, en combinaison avec au moins une thérapie anticancéreuse, pour une utilisation selon l'une quelconque des revendications 1 à 3, dans lequel la thérapie anticancéreuse est un inhibiteur de MEK choisi dans le groupe constitué du binimétinib, du cobimétinib, du sélumétinib, du tramétinib, de sels acceptables pharmaceutiquement de ceux-ci et de combinaisons de ceux-ci.

9. Composé 1, ou sel acceptable pharmaceutiquement de celui-ci, en combinaison avec au moins une thérapie anticancéreuse, pour une utilisation selon l'une quelconque des revendications 1 à 3, dans lequel la thérapie anticancéreuse est un inhibiteur d'ERK choisi dans le groupe constitué du ravoxertinib, de MK-8353, de l'ulixertinib, du temuterkib, du KO-947, du CC-90003, de l'ONC201, du tizaterkib, du SCH772984, de la pluripotine, du VX-1 le, du DEL-22379, du FR 180204, de l'ERK5-IN-1, de sels acceptables pharmaceutiquement de ceux-ci et de combinaisons de ceux-ci.

10. Composé 1, ou sel acceptable pharmaceutiquement de celui-ci, en combinaison avec au moins une thérapie anticancéreuse, pour une utilisation selon l'une quelconque des revendications 1 à 3, dans lequel la thérapie anticancéreuse est un inhibiteur de PARP1 choisi dans le groupe constitué du rucaparib, de l'iniparib, de l'olaparib, du véliparib, du niraparib, du talazoparib, du CEP-9722 et de l'E7016, de préférence, dans lequel l'inhibiteur de PARP1 est l'olaparib.

11. Composé 1, ou sel acceptable pharmaceutiquement de celui-ci, en combinaison avec au moins une thérapie anticancéreuse, pour une utilisation selon l'une quelconque des revendications 1 à 3, dans lequel la thérapie anticancéreuse est le tamoxifène.

12. Composé 1, ou sel acceptable pharmaceutiquement de celui-ci, en combinaison avec au moins une thérapie anticancéreuse, pour une utilisation selon l'une quelconque des revendications 1 à 10, dans lequel le Composé 1 et l'au moins une thérapie anticancéreuse sont administrés au sujet une fois par jour pendant 4 jours de chaque semaine, une fois par jour pendant 5 jours de chaque semaine, une fois par jour pendant 6 jours de chaque semaine ou une fois par jour pendant 7 jours de chaque semaine.

13. Composé 1, ou sel acceptable pharmaceutiquement de celui-ci, en combinaison avec au moins une thérapie anticancéreuse, pour une utilisation selon l'une quelconque des revendications 1 à 12, dans lequel la dose de la thérapie anticancéreuse ou du Composé 1 est inférieure à la dose qui serait nécessaire pour la thérapie anticancéreuse ou le Composé 1 si l'un ou l'autre devait être administré seul au sujet pour atteindre le même critère thérapeutique.

14. Composé 1, ou sel acceptable pharmaceutiquement de celui-ci, en combinaison avec au moins une thérapie anticancéreuse, pour une utilisation selon l'une quelconque des revendications 1 à 13, dans lequel le critère thérapeutique de l'administration du Composé 1 en combinaison avec au moins une thérapie anticancéreuse est une survie du sujet supérieure à celle qu'il aurait eue si le sujet avait reçu l'agent anticancéreux ou le Composé 1 seul.

15. Composé 1, ou sel acceptable pharmaceutiquement de celui-ci, en combinaison avec au moins une thérapie anticancéreuse, pour une utilisation selon l'une quelconque des revendications 1 à 14, dans lequel chaque dose de Composé 1 dans la combinaison est d'environ 0,1 à environ 15 mg/kg.
